# EUROPEAN PATENT SPECIFICATION

(11) **EP 2 931 920 B1**
(45) Date of publication and mention of the grant of the patent: **07.03.2018**
(21) Application number: 13862455.6
(22) Date of filing: 13.12.2013
(51) Int. Cl.: C12Q 1/68, G01N 33/50

(54) **BIOMARKERS OF ASTHMA INFLAMMATORY PHENOTYPES AND RESPONSE TO THERAPY**
BIOMARKER FÜR INFLAMMATORISCHE ASTHMA-PHÄNOTYPEN UND THERAPIEERFOLG
BIOMARQUEURS DE PHÉNOTYPES INFLAMMATOIRES DE L'ASTHME ET DE LA RÉPONSE À LA THÉRAPIE

(30) Priority: 14.12.2012 AU 2012905473
(43) Date of publication of application: 21.10.2015
(73) Proprietor: The University of Newcastle, Callaghan, New South Wales 2308 (AU)
(72) Inventor: BAINES, Katherine Joanne, Floraville, New South Wales 2280 (AU); GIBSON, Peter G., Merewether, New South Wales 2291 (AU)
(74) Representative: Potter Clarkson LLP
(86) International application number: PCT/AU2013/001466
(87) International publication number: WO 2014/089636

(56) References cited:
- WO-A1-2006/119343
- WO-A1-2009/124090
- WO-A2-2008/086043
- JUSTIN C. CHUA ET AL: "Galectin-10, a Potential Biomarker of Eosinophilic Airway Inflammation", PLOS ONE, vol. 7, no. 8, 6 August 2012 (2012-08-06), page e42549, XP055286634, DOI: 10.1371/journal.pone.0042549
- ZHU ZHOU ET AL: "Pulmonary expression of interleukin-13 causes inflammation, mucus hypersecretion, subepithelial fibrosis, physiologic abnormalities, and eotaxin production", JOURNAL OF CLINICAL INVESTIGATION, AMERICAN SOCIETY FOR CLINICAL INVESTIGATION, US, vol. 103, no. 6, 1 March 1999 (1999-03-01) , pages 779-788, XP002176474, ISSN: 0021-9738, DOI: 10.1172/JCI5909
- Jing Ying Ma ET AL: "Selective p38[alpha] mitogen-activated protein kinase inhibitor attenuates lung infl ammation and fi brosis in IL-13 transgenic mouse model of asthma", Journal of asthma and allergy, 1 January 2008 (2008-01-01), pages 31-44, XP055286812, Retrieved from the Internet: URL:http://www.ncbi.nlm.nih.gov/pmc/articl es/PMC3121334/pdf/jaa_4199_selective_p38a_ mitogen.pdf [retrieved on 2016-07-07]
- T X LU ET AL: "MiR-375 is downregulated in epithelial cells after IL-13 stimulation and regulates an IL-13-induced epithelial transcriptome", MUCOSAL IMMUNOLOGY, vol. 5, no. 4, 1 July 2012 (2012-07-01), pages 388-396, XP055200584, ISSN: 1933-0219, DOI: 10.1038/mi.2012.16
- KATHERINE J. BAINES ET AL: "Sputum gene expression signature of 6 biomarkers discriminates asthma inflammatory phenotypes", JOURNAL OF ALLERGY AND CLINICAL IMMUNOLOGY, vol. 133, no. 4, 1 April 2014 (2014-04-01) , pages 997-1007, XP055286629, AMSTERDAM, NL ISSN: 0091-6749, DOI: 10.1016/j.jaci.2013.12.1091
- BAINES, KJ. ET AL.: 'Transcriptional phenotypes of asthma defined by gene expression profiling of induced sputum samples' J. ALLERGY CLIN. IMMUNOL. vol. 127, 2011, pages 153 - 160, XP028150399
- BAINES, KJ. ET AL.: 'Differential gene expression and cytokine production from neutrophils in asthma phenotypes' EUR. RESPIR. J. vol. 35, 2010, pages 522 - 531, XP055258968
- DATABASE GEO [Online] 01 June 2007 'Illumina HumanRef-8 Expression BeadChip', XP055258985 Retrieved from NCBI Database accession no. GPL5060
- FU, J-J. ET AL.: 'Systemic inflammation is associated with differential gene expression and airway neutrophilia in asthma' OMICS. vol. 17, 2013, pages 187 - 199, XP055258970
- SIMPSON, JL. ET AL.: 'Elevated expression of the NLRP3 inflammasome in neutrophilic asthma' EUR. RESPIR. J. 2013, XP055258971

### Sequence Listing

<110> Newcastle Innovation Limited
<120> Biomarkers of asthma inflammatory phenotypes
<130> 35211398
<160> 16
<170> PatentIn version 3.4
<210> 1
   <211> 429
   <212> DNA
   <213> Homo sapiens
<400> 1
   atgtccctgc tacccgtgcc atacacagag gctgcctctt tgtctactgg ttctactgtg     60
   acaatcaaag ggcgaccact tgcctgtttc ttgaatgaac catatctgca ggtggatttc    120
   cacactgaga tgaaggagga atcagacatt gtcttccatt tccaagtgtg ctttggtcgt    180
   cgtgtggtca tgaacagccg tgagtatggg gcctggaagc agcaggtgga atccaagaat    240
   atgccctttc aggatggcca agaatttgaa ctgagcatct cagtgctgcc agataagtac    300
   caggtaatgg tcaatggcca atcctcttac acctttgacc atagaatcaa gcctgaggct    360
   gtgaagatgg tgcaagtgtg gagagatatc tccctgacca aatttaatgt cagctattta    420
   aagagataa                                                            429
<210> 2
   <211> 142
   <212> PRT
   <213> Homo sapiens
<400> 2
   Met Ser Leu Leu Pro Val Pro Tyr Thr Glu Ala Ala Ser Leu Ser Thr
   1               5                   10                  15
   Gly Ser Thr Val Thr Ile Lys Gly Arg Pro Leu Ala Cys Phe Leu Asn
               20                  25                  30
   Glu Pro Tyr Leu Gln Val Asp Phe His Thr Glu Met Lys Glu Glu Ser
           35                  40                  45
   Asp Ile Val Phe His Phe Gln Val Cys Phe Gly Arg Arg Val Val Met
       50                  55                  60
   Asn Ser Arg Glu Tyr Gly Ala Trp Lys Gln Gln Val Glu Ser Lys Asn
   65                  70                  75                  80
   Met Pro Phe Gln Asp Gly Gln Glu Phe Glu Leu Ser Ile Ser Val Leu
                   85                  90                  95
   Pro Asp Lys Tyr Gln Val Met Val Asn Gly Gln Ser Ser Tyr Thr Phe
               100                 105                 110
   Asp His Arg Ile Lys Pro Glu Ala Val Lys Met Val Gln Val Trp Arg
           115                 120                 125
   Asp Ile Ser Leu Thr Lys Phe Asn Val Ser Tyr Leu Lys Arg
       130                 135                 140
<210> 3
   <211> 1254
   <212> DNA
   <213> Homo sapiens
<400> 3
   atgaggctca tcctgcctgt gggtttgatt gctaccactc ttgcaattgc tcctgtccgc      60
   tttgacaggg agaaggtgtt ccgcgtgaag ccccaggatg aaaaacaagc agacatcata     120
   aaggacttgg ccaaaaccaa tgagcttgac ttctggtatc caggtgccac ccaccacgta     180
   gctgctaata tgatggtgga tttccgagtt agtgagaagg aatcccaagc catccagtct     240
   gccttggatc aaaataaaat gcactatgaa atcttgattc atgatctaca agaagagatt     300
   gagaaacagt ttgatgttaa agaagatatc ccaggcaggc acagctacgc aaaatacaat     360
   aattgggaaa agattgtggc ttggactgaa aagatgatgg ataagtatcc tgaaatggtc     420
   tctcgtatta aaattggatc tactgttgaa gataatccac tatatgttct gaagattggg     480
   gaaaagaatg aaagaagaaa ggctattttt acggattgtg gcattcacgc acgagaatgg     540
   gtctccccag cattctgcca gtggtttgtc tatcaggcaa ccaaaactta tgggagaaac     600
   aaaattatga ccaaactctt ggaccgaatg aatttttaca ttcttcctgt gttcaatgtt     660
   gatggatata tttggtcatg gacaaagaac cgcatgtgga gaaaaaatcg ttccaagaac     720
   caaaactcca aatgcatcgg cactgacctc aacaggaatt ttaatgcttc atggaactcc     780
   attcctaaca ccaatgaccc atgtgcagat aactatcggg gctctgcacc agagtccgag     840
   aaagagacga aagctgtcac taatttcatt agaagccacc tgaatgaaat caaggtttac     900
   atcaccttcc attcctactc ccagatgcta ttgtttccct atggatatac atcaaaactg     960
   ccacctaacc atgaggactt ggccaaagtt gcaaagattg gcactgatgt tctatcaact    1020
   cgatatgaaa cccgctacat ctatggccca atagaatcaa caatttaccc gatatcaggt    1080
   tcttctttag actgggctta tgacctgggc atcaaacaca catttgcctt tgagctccga    1140
   gataaaggca aatttggttt tctccttcca gaatcccgga taaagccaac gtgcagagag    1200
   accatgctag ctgtcaaatt tattgccaag tatatcctca agcatacttc ctaa          1254
<210> 4
   <211> 417
   <212> PRT
   <213> Homo sapiens
<400> 4
   Met Arg Leu Ile Leu Pro Val Gly Leu Ile Ala Thr Thr Leu Ala Ile
   1               5                   10                  15
   Ala Pro Val Arg Phe Asp Arg Glu Lys Val Phe Arg Val Lys Pro Gln
               20                  25                  30
   Asp Glu Lys Gln Ala Asp Ile Ile Lys Asp Leu Ala Lys Thr Asn Glu
           35                  40                  45
   Leu Asp Phe Trp Tyr Pro Gly Ala Thr His His Val Ala Ala Asn Met
       50                  55                  60
   Met Val Asp Phe Arg Val Ser Glu Lys Glu Ser Gln Ala Ile Gln Ser
   65                  70                  75                  80
   Ala Leu Asp Gln Asn Lys Met His Tyr Glu Ile Leu Ile His Asp Leu
                   85                  90                  95
   Gln Glu Glu Ile Glu Lys Gln Phe Asp Val Lys Glu Asp Ile Pro Gly
               100                 105                 110
   Arg His Ser Tyr Ala Lys Tyr Asn Asn Trp Glu Lys Ile Val Ala Trp
           115                 120                 125
   Thr Glu Lys Met Met Asp Lys Tyr Pro Glu Met Val Ser Arg Ile Lys
       130                 135                 140
   Ile Gly Ser Thr Val Glu Asp Asn Pro Leu Tyr Val Leu Lys Ile Gly
   145                 150                 155                 160
   Glu Lys Asn Glu Arg Arg Lys Ala Ile Phe Thr Asp Cys Gly Ile His
                   165                 170                 175
   Ala Arg Glu Trp Val Ser Pro Ala Phe Cys Gln Trp Phe Val Tyr Gln
               180                 185                 190
   Ala Thr Lys Thr Tyr Gly Arg Asn Lys Ile Met Thr Lys Leu Leu Asp
   195 200 205
   Arg Met Asn Phe Tyr Ile Leu Pro Val Phe Asn Val Asp Gly Tyr Ile
   210 215 220
   Trp Ser Trp Thr Lys Asn Arg Met Trp Arg Lys Asn Arg Ser Lys Asn
   225 230 235 240
   Gln Asn Ser Lys Cys Ile Gly Thr Asp Leu Asn Arg Asn Phe Asn Ala
   245 250 255
   Ser Trp Asn Ser Ile Pro Asn Thr Asn Asp Pro Cys Ala Asp Asn Tyr
   260 265 270
   Arg Gly Ser Ala Pro Glu Ser Glu Lys Glu Thr Lys Ala Val Thr Asn
   275 280 285
   Phe Ile Arg Ser His Leu Asn Glu Ile Lys Val Tyr Ile Thr Phe His
   290 295 300
   Ser Tyr Ser Gln Met Leu Leu Phe Pro Tyr Gly Tyr Thr Ser Lys Leu
   305 310 315 320
   Pro Pro Asn His Glu Asp Leu Ala Lys Val Ala Lys Ile Gly Thr Asp
   325 330 335
   Val Leu Ser Thr Arg Tyr Glu Thr Arg Tyr Ile Tyr Gly Pro Ile Glu
   340 345 350
   Ser Thr Ile Tyr Pro Ile Ser Gly Ser Ser Leu Asp Trp Ala Tyr Asp
   355 360 365
   Leu Gly Ile Lys His Thr Phe Ala Phe Glu Leu Arg Asp Lys Gly Lys
   370 375 380
   Phe Gly Phe Leu Leu Pro Glu Ser Arg Ile Lys Pro Thr Cys Arg Glu
   385 390 395 400
   Thr Met Leu Ala Val Lys Phe Ile Ala Lys Tyr Ile Leu Lys His Thr
   405 410 415
   Ser
<210> 5
   <211> 918
   <212> DNA
   <213> Homo sapiens
<400> 5
   atgtcacggg agctggcccc actgctgctt ctcctcctct ccatccacag cgccctggcc     60
   atgaggatct gctccttcaa cgtcaggtcc tttggggaaa gcaagcagga agacaagaat    120
   gccatggatg tcattgtgaa ggtcatcaaa cgctgtgaca tcatactcgt gatggaaatc    180
   aaggacagca acaacaggat ctgccccata ctgatggaga agctgaacag aaattcaagg    240
   agaggcataa cgtacaacta tgtgattagc tctcggcttg gaagaaacac atataaagaa    300
   caatatgcct ttctctacaa ggaaaagctg gtgtctgtga agaggagtta tcactaccat    360
   gactatcagg atggagacgc agatgtgttt tccagggagc cctttgtggt ctggttccaa    420
   tctccccaca ctgctgtcaa agacttcgtg attatccccc tgcacaccac cccagagaca    480
   tccgttaagg agatcgatga gttggttgag gtctacacgg acgtgaaaca ccgctggaag    540
   gcggagaatt tcattttcat gggtgacttc aatgccggct gcagctacgt ccccaagaag    600
   gcctggaaga acatccgctt gaggactgac cccaggtttg tttggctgat cggggaccaa    660
   gaggacacca cggtgaagaa gagcaccaac tgtgcatatg acaggattgt gcttagagga    720
   caagaaatcg tcagttctgt tgttcccaag tcaaacagtg tttttgactt ccagaaagct    780
   tacaagctga ctgaagagga ggccctggat gtcagcgacc actttccagt tgaatttaaa    840
   ctacagtctt caagggcctt caccaacagc aaaaaatctg tcactctaag gaagaaaaca    900
   aagagcaaac gctcctag                                                  918
<210> 6
   <211> 305
   <212> PRT
   <213> Homo sapiens
<400> 6
   Met Ser Arg Glu Leu Ala Pro Leu Leu Leu Leu Leu Leu Ser Ile His
   1               5                   10                  15
   Ser Ala Leu Ala Met Arg Ile Cys Ser Phe Asn Val Arg Ser Phe Gly
               20                  25                  30
   Glu Ser Lys Gln Glu Asp Lys Asn Ala Met Asp Val Ile Val Lys Val
           35                  40                  45
   Ile Lys Arg Cys Asp Ile Ile Leu Val Met Glu Ile Lys Asp Ser Asn
       50                  55                  60
   Asn Arg Ile Cys Pro Ile Leu Met Glu Lys Leu Asn Arg Asn Ser Arg
   65                  70                  75                  80
   Arg Gly Ile Thr Tyr Asn Tyr Val Ile Ser Ser Arg Leu Gly Arg Asn
                   85                  90                  95
   Thr Tyr Lys Glu Gln Tyr Ala Phe Leu Tyr Lys Glu Lys Leu Val Ser
               100                 105                 110
   Val Lys Arg Ser Tyr His Tyr His Asp Tyr Gln Asp Gly Asp Ala Asp
           115                 120                 125
   Val Phe Ser Arg Glu Pro Phe Val Val Trp Phe Gln Ser Pro His Thr
       130                 135                 140
   Ala Val Lys Asp Phe Val Ile Ile Pro Leu His Thr Thr Pro Glu Thr
   145                 150                 155                 160
   Ser Val Lys Glu Ile Asp Glu Leu Val Glu Val Tyr Thr Asp Val Lys
                   165                 170                 175
   His Arg Trp Lys Ala Glu Asn Phe Ile Phe Met Gly Asp Phe Asn Ala
               180                 185                 190
   Gly Cys Ser Tyr Val Pro Lys Lys Ala Trp Lys Asn Ile Arg Leu Arg
           195                 200                 205
   Thr Asp Pro Arg Phe Val Trp Leu Ile Gly Asp Gln Glu Asp Thr Thr
       210                 215                 220
   Val Lys Lys Ser Thr Asn Cys Ala Tyr Asp Arg Ile Val Leu Arg Gly
   225                 230                 235                 240
   Gln Glu Ile Val Ser Ser Val Val Pro Lys Ser Asn Ser Val Phe Asp
                   245                 250                 255
   Phe Gln Lys Ala Tyr Lys Leu Thr Glu Glu Glu Ala Leu Asp Val Ser
               260                 265                 270
   Asp His Phe Pro Val Glu Phe Lys Leu Gln Ser Ser Arg Ala Phe Thr
           275                 280                 285
   Asn Ser Lys Lys Ser Val Thr Leu Arg Lys Lys Thr Lys Ser Lys Arg
       290                 295                 300
   Ser
   305
<210> 7
   <211> 810
   <212> DNA
   <213> Homo sapiens
<400> 7
   atggcagaag tacctgagct cgccagtgaa atgatggctt attacagtgg caatgaggat      60
   gacttgttct ttgaagctga tggccctaaa cagatgaagt gctccttcca ggacctggac     120
   ctctgccctc tggatggcgg catccagcta cgaatctccg accaccacta cagcaagggc     180
   ttcaggcagg ccgcgtcagt tgttgtggcc atggacaagc tgaggaagat gctggttccc     240
   tgcccacaga ccttccagga gaatgacctg agcaccttct ttcccttcat ctttgaagaa      300
   gaacctatct tcttcgacac atgggataac gaggcttatg tgcacgatgc acctgtacga     360
   tcactgaact gcacgctccg ggactcacag caaaaaagct tggtgatgtc tggtccatat     420
   gaactgaaag ctctccacct ccagggacag gatatggagc aacaagtggt gttctccatg     480
   tcctttgtac aaggagaaga aagtaatgac aaaatacctg tggccttggg cctcaaggaa     540
   aagaatctgt acctgtcctg cgtgttgaaa gatgataagc ccactctaca gctggagagt     600
   gtagatccca aaaattaccc aaagaagaag atggaaaagc gatttgtctt caacaagata     660
   gaaatcaata acaagctgga atttgagtct gcccagttcc ccaactggta catcagcacc     720
   tctcaagcag aaaacatgcc cgtcttcctg ggagggacca aaggcggcca ggatataact     780
   gacttcacca tgcaatttgt gtcttcctaa                                      810
<210> 8
   <211> 269
   <212> PRT
   <213> Homo sapiens
<400> 8
   Met Ala Glu Val Pro Glu Leu Ala Ser Glu Met Met Ala Tyr Tyr Ser
   1               5                   10                  15
   Gly Asn Glu Asp Asp Leu Phe Phe Glu Ala Asp Gly Pro Lys Gln Met
               20                  25                  30
   Lys Cys Ser Phe Gln Asp Leu Asp Leu Cys Pro Leu Asp Gly Gly Ile
           35                  40                  45
   Gln Leu Arg Ile Ser Asp His His Tyr Ser Lys Gly Phe Arg Gln Ala
       50                  55                  60
   Ala Ser Val Val Val Ala Met Asp Lys Leu Arg Lys Met Leu Val Pro
   65                  70                  75                  80
   Cys Pro Gln Thr Phe Gln Glu Asn Asp Leu Ser Thr Phe Phe Pro Phe
                   85                  90                  95
   Ile Phe Glu Glu Glu Pro Ile Phe Phe Asp Thr Trp Asp Asn Glu Ala
               100                 105                 110
   Tyr Val His Asp Ala Pro Val Arg Ser Leu Asn Cys Thr Leu Arg Asp
           115                 120                 125
   Ser Gln Gln Lys Ser Leu Val Met Ser Gly Pro Tyr Glu Leu Lys Ala
       130                 135                 140
   Leu His Leu Gln Gly Gln Asp Met Glu Gln Gln Val Val Phe Ser Met
   145                 150                 155                 160
   Ser Phe Val Gln Gly Glu Glu Ser Asn Asp Lys Ile Pro Val Ala Leu
                   165                 170                 175
   Gly Leu Lys Glu Lys Asn Leu Tyr Leu Ser Cys Val Leu Lys Asp Asp
               180                 185                 190
   Lys Pro Thr Leu Gln Leu Glu Ser Val Asp Pro Lys Asn Tyr Pro Lys
           195                 200                 205
   Lys Lys Met Glu Lys Arg Phe Val Phe Asn Lys Ile Glu Ile Asn Asn
       210                 215                 220
   Lys Leu Glu Phe Glu Ser Ala Gln Phe Pro Asn Trp Tyr Ile Ser Thr
   225                 230                 235                 240
   Ser Gln Ala Glu Asn Met Pro Val Phe Leu Gly Gly Thr Lys Gly Gly
                   245                 250                 255
   Gln Asp Ile Thr Asp Phe Thr Met Gln Phe Val Ser Ser
               260                 265
<210> 9
   <211> 1575
   <212> DNA
   <213> Homo sapiens
<400> 9
   atgatttcac cattcttagt actggccatt ggcacctgcc ttactaactc cttagtgcca      60
   gagaaagaga aagaccccaa gtactggcga gaccaagcgc aagagacact gaaatatgcc     120
   ctggagcttc agaagctcaa caccaacgtg gctaagaatg tcatcatgtt cctgggagat     180
   gggatgggtg tctccacagt gacggctgcc cgcatcctca agggtcagct ccaccacaac     240
   cctggggagg agaccaggct ggagatggac aagttcccct tcgtggccct ctccaagacg     300
   tacaacacca atgcccaggt ccctgacagc gccggcaccg ccaccgccta cctgtgtggg     360
   gtgaaggcca atgagggcac cgtgggggta agcgcagcca ctgagcgttc ccggtgcaac     420
   accacccagg ggaacgaggt cacctccatc ctgcgctggg ccaaggacgc tgggaaatct     480
   gtgggcattg tgaccaccac gagagtgaac catgccaccc ccagcgccgc ctacgcccac     540
   tcggctgacc gggactggta ctcagacaac gagatgcccc ctgaggcctt gagccagggc     600
   tgtaaggaca tcgcctacca gctcatgcat aacatcaggg acattgacgt gatcatgggg     660
   ggtggccgga aatacatgta ccccaagaat aaaactgatg tggagtatga gagtgacgag     720
   aaagccaggg gcacgaggct ggacggcctg gacctcgttg acacctggaa gagcttcaaa     780
   ccgagataca agcactccca cttcatctgg aaccgcacgg aactcctgac ccttgacccc     840
   cacaatgtgg actacctatt gggtctcttc gagccagggg acatgcagta cgagctgaac     900
   aggaacaacg tgacggaccc gtcactctcc gagatggtgg tggtggccat ccagatcctg     960
   cggaagaacc ccaaaggctt cttcttgctg gtggaaggag gcagaattga ccacgggcac    1020
   catgaaggaa aagccaagca ggccctgcat gaggcggtgg agatggaccg ggccatcggg    1080
   caggcaggca gcttgacctc ctcggaagac actctgaccg tggtcactgc ggaccattcc    1140
   cacgtcttca catttggtgg atacaccccc cgtggcaact ctatctttgg tctggccccc    1200
   atgctgagtg acacagacaa gaagcccttc actgccatcc tgtatggcaa tgggcctggc    1260
   tacaaggtgg tgggcggtga acgagagaat gtctccatgg tggactatgc tcacaacaac    1320
   taccaggcgc agtctgctgt gcccctgcgc cacgagaccc acggcgggga ggacgtggcc    1380
   gtcttctcca agggccccat ggcgcacctg ctgcacggcg tccacgagca gaactacgtc    1440
   ccccacgtga tggcgtatgc agcctgcatc ggggccaacc tcggccactg tgctcctgcc    1500
   agctcggcag gcagccttgc tgcaggcccc ctgctgctcg cgctggccct ctaccccctg    1560
   agcgtcctgt tctga                                                     1575
<210> 10
   <211> 524
   <212> PRT
   <213> Homo sapiens
<400> 10 500 505 510
   Leu Ala Leu Ala Leu Tyr Pro Leu Ser Val Leu Phe 515 520
<210> 11
   <211> 1083
   <212> DNA
   <213> Homo sapiens
<400> 11
   atggaagatt ttaacatgga gagtgacagc tttgaagatt tctggaaagg tgaagatctt      60
   agtaattaca gttacagctc taccctgccc ccttttctac tagatgccgc cccatgtgaa     120
   ccagaatccc tggaaatcaa caagtatttt gtggtcatta tctatgccct ggtattcctg     180
   ctgagcctgc tgggaaactc cctcgtgatg ctggtcatct tatacagcag ggtcggccgc     240
   tccgtcactg atgtctacct gctgaaccta gccttggccg acctactctt tgccctgacc     300
   ttgcccatct gggccgcctc caaggtgaat ggctggattt ttggcacatt cctgtgcaag     360
   gtggtctcac tcctgaagga agtcaacttc tatagtggca tcctgctact ggcctgcatc     420
   agtgtggacc gttacctggc cattgtccat gccacacgca cactgaccca gaagcgctac     480
   ttggtcaaat tcatatgtct cagcatctgg ggtctgtcct tgctcctggc cctgcctgtc     540
   ttacttttcc gaaggaccgt ctactcatcc aatgttagcc cagcctgcta tgaggacatg     600
   ggcaacaata cagcaaactg gcggatgctg ttacggatcc tgccccagtc ctttggcttc     660
   atcgtgccac tgctgatcat gctgttctgc tacggattca ccctgcgtac gctgtttaag     720
   gcccacatgg ggcagaagca ccgggccatg cgggtcatct ttgctgtcgt cctcatcttc     780
   ctgctctgct ggctgcccta caacctggtc ctgctggcag acaccctcat gaggacccag     840
   gtgatccagg agacctgtga gcgccgcaat cacatcgacc gggctctgga tgccaccgag     900
   attctgggca tccttcacag ctgcctcaac cccctcatct acgccttcat tggccagaag     960
   tttcgccatg gactcctcaa gattctagct atacatggct tgatcagcaa ggactccctg    1020
   cccaaagaca gcaggccttc ctttgttggc tcttcttcag ggcacacttc cactactctc    1080
   taa                                                                  1083
<210> 12
   <211> 360
   <212> PRT
   <213> Homo sapiens
<400> 12
<210> 13
   <211> 1989
   <212> DNA
   <213> Homo sapiens
<400> 13
   atgatggagc ctgaggagta cagagagaga gggagagaga tggtggatta catctgccag 60
   tacctgagca ctgtgcggga gagacgtgtg acgccagacg tgcagcctgg ctacctgcga 120
   gcccagctgc ctgagagtgc tcctgaggac cccgacagct gggacagcat ctttggggac 180
   attgaacgaa tcatcatgcc tggggtggta cattggcaga gcccccatat gcacgcctac 240
   tacccagccc tcacctcttg gccctccctg ctaggagaca tgctggctga tgccatcaac 300
   tgcttgggat tcacctgggc atccagccct gcgtgtacag agctggagat gaacgtcatg 360
   gactggttgg caaaaatgct gggacttcca gagcacttct tgcaccacca ccccagcagc 420
   cagggcggag gcgtcctgca gagcacggtc agtgaatcca ctttgattgc cctgctggca 480
   gcaaggaaga acaaaatcct ggaaatgaaa acgtctgagc ccgatgctga tgagtcctgc 540
   ctaaatgccc gactcgtggc ctatgcctct gaccaggctc actcctctgt ggaaaaggct 600
   ggtttgattt cccttgtgaa gatgaaattt ctgcctgtgg atgacaactt ctcactccga 660
   ggggaagctc ttcagaaggc catcgaggaa gacaagcagc ggggcttggt gcccgtcttt 720
   gtctgtgcaa cactagggac cactggggtc tgtgcatttg actgcctgtc agagctgggc 780
   cccatctgtg cccgtgaggg gctgtggctc cacatcgatg ctgcttatgc aggcactgcc    840
   ttcctgtgcc ccgagttccg ggggtttctg aaggggattg agtatgccga ctccttcacc    900
   tttaatcctt ccaagtggat gatggtgcat tttgactgta ctgggttctg ggtcaaggac    960
   aagtacaagc tgcagcagac cttcagtgtg aatcccatct acctcaggca tgccaactca   1020
   ggcgtggcca ccgacttcat gcactggcag atccccctga gccgacggtt tcgctctgtt   1080
   aaactctggt tcgtgattcg gtccttcggg gtgaagaatc ttcaagcaca tgtcagacat   1140
   ggtactgaaa tggctaaata ttttgaatct ctggtcagaa acgacccttc ctttgaaatt   1200
   cctgccaaga ggcaccttgg cctggtggtt tttcgtctaa agggtcctaa ttgtctcaca   1260
   gaaaatgtgt taaaggaaat agctaaagct ggccgtctct tcctcatccc ggccactatc   1320
   caggacaagt taatcatccg tttcactgtg acatcccagt ttaccactag ggatgacatc   1380
   ctgagagact ggaatctcat tcgagatgct gccactctca tcctgagtca gcactgtact   1440
   tcccaaccca gccctcgggt tgggaacctc atctcccaaa tcaggggtgc cagagcctgg   1500
   gcctgtggaa cgtcccttca gtctgtcagt ggggcaggag atgatccagt ccaggccagg   1560
   aagatcatca agcagcctca gcgtgtggga gccggtccca tgaaaaggga aaatggcctc   1620
   catcttgaaa ccctgctgga cccagttgat gactgctttt cagaagaggc cccagatgcc   1680
   accaagcaca agctgtcctc cttcctgttc agttacttgt ctgtgcagac taagaagaag   1740
   acggtgcgct ccctcagttg caacagtgtg ccagtgagtg ctcagaagcc actgcccaca   1800
   gaggcctctg tgaagaatgg gggctcctcc agggtcagaa tcttttccag gtttccagaa   1860
   gacatgatga tgctgaagaa aagtgccttc aaaaaactca tcaaattcta cagcgtcccc   1920
   agctttcctg aatgcagctc tcaatgtgga ctccagctgc cctgttgccc tctgcaggcc   1980
   atggtttag                                                           1989
<210> 14
   <211> 662
   <212> PRT
   <213> Homo sapiens
<400> 14
   Met Met Glu Pro Glu Glu Tyr Arg Glu Arg Gly Arg Glu Met Val Asp
   1               5                   10                  15
   Tyr Ile Cys Gln Tyr Leu Ser Thr Val Arg Glu Arg Arg Val Thr Pro
               20                  25                  30
   Asp Val Gln Pro Gly Tyr Leu Arg Ala Gln Leu Pro Glu Ser Ala Pro
           35                  40                  45
   Glu Asp Pro Asp Ser Trp Asp Ser Ile Phe Gly Asp Ile Glu Arg Ile
       50                  55                  60
   Ile Met Pro Gly Val Val His Trp Gln Ser Pro His Met His Ala Tyr
   65                  70                  75                  80
   Tyr Pro Ala Leu Thr Ser Trp Pro Ser Leu Leu Gly Asp Met Leu Ala
                   85                  90                  95
   Asp Ala Ile Asn Cys Leu Gly Phe Thr Trp Ala Ser Ser Pro Ala Cys
               100                 105                 110
   Thr Glu Leu Glu Met Asn Val Met Asp Trp Leu Ala Lys Met Leu Gly
           115                 120                 125
   Leu Pro Glu His Phe Leu His His His Pro Ser Ser Gln Gly Gly Gly
       130                 135                 140
   Val Leu Gln Ser Thr Val Ser Glu Ser Thr Leu Ile Ala Leu Leu Ala
   145                 150                 155                 160
   Ala Arg Lys Asn Lys Ile Leu Glu Met Lys Thr Ser Glu Pro Asp Ala
                   165                 170                 175
   Asp Glu Ser Cys Leu Asn Ala Arg Leu Val Ala Tyr Ala Ser Asp Gln
               180                 185                 190
   Ala His Ser Ser Val Glu Lys Ala Gly Leu Ile Ser Leu Val Lys Met
           195                 200                 205
   Lys Phe Leu Pro Val Asp Asp Asn Phe Ser Leu Arg Gly Glu Ala Leu
       210                 215                 220
   Gln Lys Ala Ile Glu Glu Asp Lys Gln Arg Gly Leu Val Pro Val Phe
   225                 230                 235                 240
   Val Cys Ala Thr Leu Gly Thr Thr Gly Val Cys Ala Phe Asp Cys Leu
                   245                 250                 255
   Ser Glu Leu Gly Pro Ile Cys Ala Arg Glu Gly Leu Trp Leu His Ile
               260                 265                 270
   Asp Ala Ala Tyr Ala Gly Thr Ala Phe Leu Cys Pro Glu Phe Arg Gly
           275                 280                 285
   Phe Leu Lys Gly Ile Glu Tyr Ala Asp Ser Phe Thr Phe Asn Pro Ser
   290 295 300
   Lys Trp Met Met Val His Phe Asp Cys Thr Gly Phe Trp Val Lys Asp
   305 310 315 320
   Lys Tyr Lys Leu Gln Gln Thr Phe Ser Val Asn Pro Ile Tyr Leu Arg
   325 330 335
   His Ala Asn Ser Gly Val Ala Thr Asp Phe Met His Trp Gln Ile Pro
   340 345 350
   Leu Ser Arg Arg Phe Arg Ser Val Lys Leu Trp Phe Val Ile Arg Ser
   355 360 365
   Phe Gly Val Lys Asn Leu Gln Ala His Val Arg His Gly Thr Glu Met
   370 375 380
   Ala Lys Tyr Phe Glu Ser Leu Val Arg Asn Asp Pro Ser Phe Glu Ile
   385 390 395 400
   Pro Ala Lys Arg His Leu Gly Leu Val Val Phe Arg Leu Lys Gly Pro
   405 410 415
   Asn Cys Leu Thr Glu Asn Val Leu Lys Glu Ile Ala Lys Ala Gly Arg
   420 425 430
   Leu Phe Leu Ile Pro Ala Thr Ile Gln Asp Lys Leu Ile Ile Arg Phe
   435 440 445
   Thr Val Thr Ser Gln Phe Thr Thr Arg Asp Asp Ile Leu Arg Asp Trp
   450 455 460
   Asn Leu Ile Arg Asp Ala Ala Thr Leu Ile Leu Ser Gln His Cys Thr
   465 470 475 480
   Ser Gln Pro Ser Pro Arg Val Gly Asn Leu Ile Ser Gln Ile Arg Gly
   485 490 495
   Ala Arg Ala Trp Ala Cys Gly Thr Ser Leu Gln Ser Val Ser Gly Ala
   500 505 510
   Gly Asp Asp Pro Val Gln Ala Arg Lys Ile Ile Lys Gln Pro Gln Arg
   515 520 525
   Val Gly Ala Gly Pro Met Lys Arg Glu Asn Gly Leu His Leu Glu Thr
   530 535 540
   Leu Leu Asp Pro Val Asp Asp Cys Phe Ser Glu Glu Ala Pro Asp Ala
   545 550 555 560
   Thr Lys His Lys Leu Ser Ser Phe Leu Phe Ser Tyr Leu Ser Val Gln
   565 570 575
   Thr Lys Lys Lys Thr Val Arg Ser Leu Ser Cys Asn Ser Val Pro Val
   580 585 590
   Ser Ala Gln Lys Pro Leu Pro Thr Glu Ala Ser Val Lys Asn Gly Gly
   595 600 605
   Ser Ser Arg Val Arg Ile Phe Ser Arg Phe Pro Glu Asp Met Met Met
   610 615 620
   Leu Lys Lys Ser Ala Phe Lys Lys Leu Ile Lys Phe Tyr Ser Val Pro
   625 630 635 640
   Ser Phe Pro Glu Cys Ser Ser Gln Cys Gly Leu Gln Leu Pro Cys Cys
   645 650 655
   Pro Leu Gln Ala Met Val
   660
<210> 15
   <211> 354
   <212> DNA
   <213> Homo sapiens
<400> 15
   atgagggcca gcagcttctt gatcgtggtg gtgttcctca tcgctgggac gctggttcta 60
   gaggcagctg tcacgggagt tcctgttaaa ggtcaagaca ctgtcaaagg ccgtgttcca 120
   ttcaatggac aagatcccgt taaaggacaa gtttcagtta aaggtcaaga taaagtcaaa 180
   gcgcaagagc cagtcaaagg tccagtctcc actaagcctg gctcctgccc cattatcttg 240
   atccggtgcg ccatgttgaa tccccctaac cgctgcttga aagatactga ctgcccagga 300
   atcaagaagt gctgtgaagg ctcttgcggg atggcctgtt tcgttcccca gtga 354
<210> 16
   <211> 117
   <212> PRT
   <213> Homo sapiens
<400> 16
   Met Arg Ala Ser Ser Phe Leu Ile Val Val Val Phe Leu Ile Ala Gly
   1               5                   10                  15
   Thr Leu Val Leu Glu Ala Ala Val Thr Gly Val Pro Val Lys Gly Gln
               20                  25                  30
   Asp Thr Val Lys Gly Arg Val Pro Phe Asn Gly Gln Asp Pro Val Lys
           35                  40                  45
   Gly Gln Val Ser Val Lys Gly Gln Asp Lys Val Lys Ala Gln Glu Pro
       50                  55                  60
   Val Lys Gly Pro Val Ser Thr Lys Pro Gly Ser Cys Pro Ile Ile Leu
   65                  70                  75                  80
   Ile Arg Cys Ala Met Leu Asn Pro Pro Asn Arg Cys Leu Lys Asp Thr
                   85                  90                  95
   Asp Cys Pro Gly Ile Lys Lys Cys Cys Glu Gly Ser Cys Gly Met Ala
               100                 105                 110
   Cys Phe Val Pro Gln
           115

## Description

### Technical Field

The present invention relates generally to the use of biomarkers for discriminating between inflammatory phenotypes of asthma, based on differential expression profiles of such biomarkers. Also provided are methods for predicting the efficacy of treatments for asthma, and in particular for determining or predicting the responsiveness of subjects to corticosteroid treatment.

### Background

Asthma is the most widespread chronic health problem in the Western world and is increasing in prevalence around the world. In Australia alone, it affects over 2 million individuals. Worldwide bronchial asthma is the most common chronic disease in childhood with an overall prevalence between 5 and 20 percent across all ages. The pathogenesis is suggested to be complex and is centred on an aberrant immune response to inhalant allergens, most commonly house dust mite (HDM) allergens, that results in an inflammation of the airway wall together with an episodic constriction of the airways resulting in symptoms such as shortness of breath, wheezing, coughing, and life-threatening dyspnoea.

Asthma is a common disease of the airways that is characterised by airway hyperresponsiveness, reversible airway obstruction and episodic respiratory symptoms that results from genetic and environmental risk factors. The pathogenesis of asthma is complex with heterogenous airway inflammation and remodelling that underlies the development of different phenotypes of the disease. Responsiveness to the current available treatments is variable, with a substantial amount of patients having symptoms refractory to complex treatment regimens including high doses of inhaled or oral corticosteroids (Ito *et al*., 2006). Thus improvements in asthma diagnosis and management will only arise from a better understanding of disease heterogeneity and the development of tools for phenotype recognition to guide personalised treatment approaches.

With recognition that airway inflammation in asthma is inherently heterogeneous the cytological analysis of induced sputum has enabled the description of eosinophilic and non-eosinophilic asthma (NEA) inflammatory phenotypes (see, for example, Gibson *et al.,* 2001), with each phenotype comprising approximately 50% of total cases. Further, an examination of the percentage levels of induced sputum neutrophils alongside eosinophils has facilitated the description of four distinct inflammatory phenotypes: eosinophilic asthma (EA), neutrophilic asthma (NA), mixed granulocytic asthma (MGA), in which levels of both eosinophils and neutrophils are elevated, and paucigranulocytic asthma (PGA), in which granulocyte levels are within the normal range (Simpson *et al*., 2006).

Biomarkers of inflammation, particularly those capable of distinguishing between sputum inflammatory phenotypes, have great potential value as diagnostic tools. Currently, bronchoscopy and sputum induction are the most accurate techniques for discrimination between the four inflammatory phenotypes. The analysis of lung tissue by bronchoscopy is invasive and requires specialized expertise. Sputum induction, however, is less invasive and, although not technically demanding, requires a certain degree of expertise and cross-centre standardization. Consequently, this technique has not been implemented on a wide scale.

Exhaled biomarkers, such as fractional exhaled nitric oxide (F_{E}NO) and exhaled breath condensate (EBC), are non-invasive direct markers of airway inflammation. However, neither F_{E}NO or EBC are capable of discriminating between each of the inflammatory phenotypes and studies investigating the potential application of F_{E}NO, in combination with EBC pH, to predict inflammatory phenotype have demonstrated limited success (e.g. Hillas *et al*., 2011).

Airway inflammation, the precursor to symptom development and alterations in lung function in asthma, is essentially the basis for treatment. Consequently, a knowledge of the inflammatory phenotype of an individual patient is advantageous as it has potential impact on treatment options and long-term clinical management. Corticosteroid treatment suppresses eosinophilic inflammation, thus the presence of airway eosinophilia may indicate non-adherence to corticosteroid therapy or insufficient dosing. Patients with NEA, however, respond poorly to inhaled corticosteroids (ICS) and add-on therapies which target neutrophilic inflammation, such as macrolides, show potential in this patient population. As newer therapies for asthma focus on increasingly specific mechanistic pathways, identification of different forms of asthma will assume greater importance. This concept has been demonstrated in a randomised trial by Green *et al*. (2002), whereby basing asthma treatment on sputum eosinophils, a 50% reduction in asthma exacerbations was observed. This important effect is all the more significant since it was seen over and above current best practice using British Thoracic Society guidelines. In order to capitalise on this new knowledge of heterogeneity in asthma, measurements that are simple, repeatable, and have good performance characteristics are needed.

Justin C. Chua ET AL: PLoS ONE, vol. 7, no. 8, 6 August 2012 page e42549, DOI:10.1371/ journal.pone.0042549 discloses that Galectin-10 (CLC) is a potential biomarker for eosinophilic airway inflammation. In particular there is a correlation between the level of CLC in sputum and eosinophils in sputum.

ZHU ZHOU ET AL: , JOURNAL OF CLINICAL INVESTIGATION, AMERICAN SOCIETY FOR CLINICAL INVESTIGATION, US, vol. 103(6), 1 March 1999, pages 779-788, ISSN: 0021-9738, DOI: 10.1172/JCI5909 discloses that 317 genes from resting neutrophils are identified as exhibiting significantly different levels of expression between asthma phenotypes, in particular eosinophilic and non-eosinophilic asthma, and 221 genes are differentially expressed in induced neutrophils

It is now increasingly acknowledged that a systemic inflammatory component to asthma exists. Blood collection is minimally invasive and sample processing procedures are highly standardized. Thus, blood based biomarkers would be highly desirable for routine use in asthma and the identification of circulating biomarkers, which enable inflammatory phenotype classification, is important.

### Summary of the Disclosure

In a first aspect, the present disclosure provides a method for determining the asthma inflammatory phenotype in a subject suffering from asthma, the method comprising:
(a) isolating a biological sample from the subject; and
(b) determining the expression profile of the genes *CLC*, *CPA3*, *DNASE1L3*, *IL1B*, *ALPL* and *IL8RB*, wherein the expression profile comprises or is based on the expression levels of said genes in the sample,
wherein the expression profile of said genes is indicative of the asthma inflammatory phenotype.

Typically the expression profile of said genes and the determination of inflammatory phenotype is based on multiple logistic regression analysis of the gene expression levels.

Typically the biological sample comprises sputum. Typically the sputum is induced sputum. The sputum may be induced using hypertonic saline.

In an embodiment, the method enables the determination of whether the subject has eosinophilic asthma or non-eosinophilic asthma.

In an embodiment, multiple logistic regression analysis of the expression profile of said genes facilitates the distinction between:
(i) eosinophilic asthma and non-eosinophilic asthma, typically where non-eosinophilic asthma is neutrophilic asthma or paucigranulocytic asthma;
(ii) neutrophilic asthma and non-neutrophilic asthma, typically where non-neutrophilic asthma is eosinophilic asthma or paucigranulocytic asthma;
(iii) neutrophilic asthma and paucigranulocytic asthma;
(iv) paucigranulocytic asthma and non-paucigranulocytic asthma, typically where non-paucigranulocytic asthma is neutrophilic asthma or eosinophilic asthma; or
(v) eosinophilic asthma and neutrophilic asthma.

Typically the expression profile of the genes in the sample from the subject is compared to a reference expression profile, wherein the reference expression profile comprises an expression profile of the same genes derived from one or more individuals known not to suffer from asthma, or alternatively known to have a particular asthma inflammatory phenotype.

For determination of the expression profile, expression may be measured at the messenger ribonucleic acid (mRNA) or gene level, or at the polypeptide or protein level.

In a second aspect, the present disclosure provides a method for distinguishing between eosinophilic asthma and non-eosinophilic asthma in a subject, the method comprising:
(a) isolating a biological sample from the subject; and
(b) determining the expression profile of the genes *CLC*, *CPA3*, *DNASE1L3*, *IL1B*, *ALPL* and *IL8RB*, based on the expression levels of said genes in the sample,
wherein the expression profile of said genes is indicative of the subject having eosinophilic asthma or non-eosinophilic asthma.

In a third aspect, the present disclosure provides a method for determining the response of a subject having asthma to corticosteroid therapy, the method comprising the steps of:
(a) obtaining a biological sample from the subject and determining the expression levels of the genes *CLC*, *CPA3*, *DNASE1L3*, *IL1B* and *ALPL* in the sample;
(b) administering a corticosteroid to the subject; and
(c) obtaining a further biological sample from the subject and determining the expression levels of the genes *CLC*, *CPA3*, *DNASE1L3*, *IL1B* and *ALPL* in the sample;
wherein a reduction in expression of one or more *of CLC*, *CPA3* and *DNASE1L3* and/or an increase in expression of one or more of *IL1B* and *ALPL* following administration step (b) is indicative that the subject is responsive to corticosteroid therapy.

In a fourth aspect, the present disclosure provides a method for diagnosing responders to corticosteroid therapy for treating asthma, the method comprising the steps of:
(a) obtaining a biological sample from a subject;
(b) determining the expression profile of the genes *CLC*, *CPA3*, *DNASE1L3*, *IL1B*, *ALPL* and *IL8RB,* wherein the expression profile comprises or is based on the expression levels of said genes in the sample; and
(c) comparing the gene expression profile from the sample with a reference gene expression profile indicative of responsiveness to corticosteroid therapy, and/or to a reference gene expression profile indicative of non-responsiveness to corticosteroid therapy,
wherein similarity in expression profiles between the sample and reference profiles is indicative of the subject being a responder or non-responder to corticosteroid therapy.

A reference expression profile indicative of responsiveness to corticosteroid therapy is typically obtained from one or more individuals responsive to corticosteroid therapy. A reference expression profile indicative of non-responsiveness to corticosteroid therapy is typically obtained from one or more individuals non-responsive to corticosteroid therapy.

Disclosed outside the scope of the invention is a method for distinguishing eosinophilic asthma from non-eosinophilic, paucigranulocytic or neutrophilic asthma in a subject, the method comprising:
(a) isolating a biological sample from the subject; and
(b) determining the expression profiles of one or more genes selected from *CLC*, *CPA3*, *DNASE1L3* and *HDC*, wherein the expression profiles of the genes comprise or are based on the expression levels of the genes in the sample,
wherein the expression profiles of one or more of said genes is indicative of the subject having (i) eosinophilic asthma or non-eosinophilic asthma, (ii) eosinophilic asthma or paucigranulocytic asthma, or (iii) eosinophilic asthma or neutrophilic asthma.

Disclosed outside the scope of the invention is a method for distinguishing neutrophilic asthma from non-neutrophilic, paucigranulocytic or eosinophilic asthma in a subject, the method comprising:
(a) isolating a biological sample from the subject; and
(b) determining the expression profiles of one or more genes selected from *ALPL*, *IL1B*, *IL8RB* and *PI3*, wherein the expression profiles of the genes comprise or are based on the expression levels of the genes in the sample,
wherein the expression profiles of one or more of said genes is indicative of the subject having (i) neutrophilic asthma or non-neutrophilic asthma, (ii) neutrophilic asthma or paucigranulocytic asthma, or (iii) neutrophilic asthma or eosinophilic asthma.

In a further aspect of the present disclosure there is provided a method for determining a treatment regime for a subject suffering from asthma, the method comprising determining the asthma inflammatory phenotype in the subject in accordance with a method of one or more of the above identified aspects, and selecting an appropriate treatment regime for the subject on the basis of the determination.

### Brief Description of the Drawings

Embodiments of the present disclosure are described, by way of example only, with reference to the following drawings.
Figure 1. Receiver operating characteristic curves demonstrating that the 6 gene biomarker signature (Example 4) identifies eosinophilic from neutrophilic asthma (A), eosinophilic from paucigranulocytic asthma (B) and neutrophilic from paucigranulocytic asthma (C).
**Figure 2****.** Receiver operating characteristic curves demonstrating that the 6 gene biomarker signature (Example 4) identifies eosinophilic (A) and neutrophilic (B) asthma from healthy controls.
**Figure 3****.** Relative gene expression levels of A) *CLC*, B) *CPA3*, C) *DNASE1L3*, D) *IL1B*, E) *ALPL* and F) *IL8RB* in induced sputum samples from subjects with inflammatory phenotypes of asthma compared to healthy controls. Gene expression is calculated relative to β-actin and expressed as a fold change from the healthy control group mean. *p<0.05 versus healthy controls, #p<0.05 ^p<0.05.
**Figure 4****.** Receiver operating characteristic curves demonstrating that the 6 gene biomarker signature (Example 4) identifies ICS treatment responsiveness determined by A) a 12% change in FEV₁, B) a 0.5 point decreased in ACQ score and C) a 40% reduction in F_{E}NO.
**Figure 5****.** Relative gene expression levels of A) *CLC*, B) *CPA3*, C) *DNASE1L3*, *D) IL1B*, E) *ALPL* and F) *IL8RB* in induced sputum samples from subjects with eosinophilic or non-eosinophilic asthma before and after corticosteroid treatment. Gene expression is calculated relative to β-actin and expressed as a fold change from the healthy control group mean. *p<0.05 versus healthy controls, #p<0.05 versus non-eosinophilic asthma before ICS.

The subject specification contains amino acid and nucleotide sequence information prepared using the programme Patentln Version 3.4, presented herein in a Sequence Listing. The nucleotide sequences of the human *CLC*, *CPA3*, *DNASE1L3*, *IL1B*, *ALPL, IL8RB*, *HDC* and *PI3* genes are provided in SEQ ID NOs:1, 3, 5, 7, 9, 11, 13 and 15 respectively. The amino acid sequences of the polypeptides encoded by these genes are provided in SEQ ID NOs:2, 4, 6, 8, 10, 12, 14 and 16 respectively.

### Detailed Description

Throughout this specification and the claims which follow, unless the context requires otherwise, the word "comprise", and variations such as "comprises" or "comprising", will be understood to imply the inclusion of a stated integer or step or group of integers or steps but not the exclusion of any other integer or step or group of integers or steps.

The articles "a" and "an" are used herein to refer to one or to more than one (*i.e.,* to at least one) of the grammatical object of the article. By way of example, "an element" means one element or more than one element.

"*CLC*" refers to the gene encoding the Charcot-Leyden crystal protein. Whilst the present disclosure typically refers to the gene and polypeptide as found in humans, or to derivatives, fragments or variants thereof, those skilled in the art will appreciate that homologues of human from other species are also contemplated and encompassed. The cDNA encoding human *CLC* is located in the National Center for Biotechnology Information (NCBI) database as Accession No. NM_001828.5. An exemplary nucleotide sequence of human *CLC* is set forth in SEQ ID NO:1, and an exemplary encoded polypeptide sequence is set forth in SEQ ID NO:2.

*"CPA3"* refers to the gene encoding carboxypeptidase A3. Whilst the present disclosure typically refers to the gene and polypeptide as found in humans, or to derivatives, fragments or variants thereof, those skilled in the art will appreciate that homologues of human from other species are also contemplated and encompassed. The cDNA encoding human *CPA3* is located in the National Center for Biotechnology Information (NCBI) database as Accession No. NM_001870.2. An exemplary nucleotide sequence of human *CPA3* is set forth in SEQ ID NO:3, and an exemplary encoded polypeptide sequence is set forth in SEQ ID NO:4.

"*DNASE1L3*" refers to the gene encoding deoxyribonuclease I-like 3. Whilst the present disclosure typically refers to the gene and polypeptide as found in humans, or to derivatives, fragments or variants thereof, those skilled in the art will appreciate that homologues of human from other species are also contemplated and encompassed. The cDNA encoding human *DNASE1L3* is located in the National Center for Biotechnology Information (NCBI) database as Accession No. NM_004944.3. An exemplary nucleotide sequence of human *DNASE1L3* is set forth in SEQ ID NO:5, and an exemplary encoded polypeptide sequence is set forth in SEQ ID NO:6.

"*IL1B*" refers to the gene encoding interleukin-1β. Whilst the present disclosure typically refers to the gene and polypeptide as found in humans, or to derivatives, fragments or variants thereof, those skilled in the art will appreciate that homologues of human from other species are also contemplated and encompassed. The cDNA encoding human *IL1B* is located in the National Center for Biotechnology Information (NCBI) database as Accession No. NM_000576.2. An exemplary nucleotide sequence of human *IL1B* is set forth in SEQ ID NO:7, and an exemplary encoded polypeptide sequence is set forth in SEQ ID NO:8.

*"ALPL"* refers to the gene encoding alkaline phosphatase, tissue-nonspecific isozyme. Whilst the present disclosure typically refers to the gene and polypeptide as found in humans, or to derivatives, fragments or variants thereof, those skilled in the art will appreciate that homologues of human from other species are also contemplated and encompassed. The cDNA encoding human *ALPL* is located in the National Center for Biotechnology Information (NCBI) database as Accession No. NM_000478.4. An exemplary nucleotide sequence of human *ALPL* is set forth in SEQ ID NO:9, and an exemplary encoded polypeptide sequence is set forth in SEQ ID NO:10.

"*IL8RB*" refers to the gene encoding interleukin-8 receptor B, also known as *CXCR2* (chemokine (C-X-C motif) receptor 2). Reference herein to *IL8RB* should be understood to be a reference to *CXCR2.* Whilst the present disclosure typically refers to the gene and polypeptide as found in humans, or to derivatives, fragments or variants thereof, those skilled in the art will appreciate that homologues of human from other species are also contemplated and encompassed. The cDNA encoding human *IL8RB* is located in the National Center for Biotechnology Information (NCBI) database as Accession No. NM_001557.3. An exemplary nucleotide sequence of human *IL8RB* is set forth in SEQ ID NO:11, and an exemplary encoded polypeptide sequence is set forth in SEQ ID NO:12.

"*HDC*" refers to the gene encoding histidine decarboxylase C. Whilst the present disclosure typically refers to the gene and polypeptide as found in humans, or to derivatives, fragments or variants thereof, those skilled in the art will appreciate that homologues of human from other species are also contemplated and encompassed. The cDNA encoding human *HDC* is located in the National Center for Biotechnology Information (NCBI) database as Accession No. NM_002112.3. An exemplary nucleotide sequence of human *HDC* is set forth in SEQ ID NO:13, and an exemplary encoded polypeptide sequence is set forth in SEQ ID NO:14.

"*PI3*" refers to the gene encoding peptidase inhibitor 3. Whilst the present disclosure typically refers to the gene and polypeptide as found in humans, or to derivatives, fragments or variants thereof, those skilled in the art will appreciate that homologues of human from other species are also contemplated and encompassed. The cDNA encoding human *PI3* is located in the National Center for Biotechnology Information (NCBI) database as Accession No. NM_002638.3. An exemplary nucleotide sequence of human *PI3* is set forth in SEQ ID NO:15, and an exemplary encoded polypeptide sequence is set forth in SEQ ID NO:16.

As used herein the term "gene" means a nucleic acid molecule having a particular function. As such the term "gene" encompasses not only the genomic nucleic acid molecule, but also the mRNA product of the genomic molecule, and equivalent cDNA molecules, as well as functionally equivalent genomic variants, alternative splicing variants and genetic isoforms of the gene.

As used herein the term "protein" means a peptide or polypeptide molecule having a particular function. As such the term "protein" encompasses not only the peptide or polypeptide product of a gene, but also functionally equivalent fragments, derivatives and variants thereof and post-translationally modified forms of the peptide or polypeptide product. Different isoforms of a protein are also encompassed by this general term. Also encompassed by the term "protein" as used herein are mature protein and polypeptide sequences, in addition to proproteins, preproproteins and other precursor molecules including, for example, signal peptides, activation peptides or other sequences cleaved from a precursor molecule to generate the mature protein or polypeptide sequence.

As used herein the term "expression profile" may refer to the expression level of one or more genes or proteins in a given sample or to a value determined from the expression level of one or more genes or proteins. Such a value may be determined by statistical analysis of expression levels as described herein. The expression profile of a group or set of two or more genes or proteins may be referred to herein as a 'combined expression profile'. Expression levels of genes and proteins may be measured for example by any suitable method for determining, and typically quantifying, gene and protein expression known to those skilled in the art. The person of skill in the art can determine the most appropriate means of analysis in any given situation.

In the context of the present disclosure, reference to an increase or decrease in an expression profile or combined expression profile in a given sample means an increase or decrease in the level of expression of the gene(s) or protein(s) in question in the sample, typically when compared to the expression levels in one or more control samples. In some embodiments an increase or decrease in an expression profile or combined expression profile in a given sample obtained from a subject following therapy or treatment may refer to an increase or decrease in the level of expression of the gene(s) or protein(s) in question when compared to the expression levels prior to, or in the absence of, said therapy or treatment.

The expression profile employed in diagnostic methods disclosed herein may be subject to, or may result from statistical analysis of expression levels, for example, in comparing expression profiles between samples including control samples. Statistical techniques that may be employed for such analyses are known to those skilled in the art and include, but are not limited to, meta-analysis, multiple regression analysis, and receiver operator curve (ROC) analysis. ROC analysis is used to determine a score that is diagnostic with the greatest sensitivity and specificity. The 'squarer' the look of the curve, the simpler the determination of a diagnostic level or score. The closer the area under the curve is to 1 also indicates a result with high sensitivity and specificity.

The term "subject" as used herein refers to mammals and includes humans, primates, livestock animals (eg. sheep, pigs, cattle, horses, donkeys), laboratory test animals (eg. mice, rabbits, rats, guinea pigs), companion animals (eg. dogs, cats) and captive wild animals (eg. foxes, kangaroos, deer). Typically, the mammal is human or a laboratory test animal. Even more typically, the mammal is a human.

As used herein the term "treatment" refers to any and all treatments that remedy a condition or one or more symptoms of a condition or disease, prevent the establishment of a condition or disease, or otherwise prevent, hinder, retard, or reverse the progression of a condition or disease or other undesirable symptoms in any way whatsoever. Thus the term "treatment" is to be considered in its broadest context. For example, treatment does not necessarily imply that a patient is treated until total recovery.

Effective clinical management of asthma requires objective measurement of inflammatory phenotype. However, the most direct measures of airway inflammation are too invasive and have limited clinical use. By using the genome wide expression analysis described herein the present inventors have identified that the combined expression profile of specific genes can distinguish between inflammatory phenotypes of asthma, and thus can also be employed to predict and monitor patient response to therapeutic intervention.

Specifically, as exemplified herein, the inventors have identified genes capable of serving as non-invasive discriminatory biomarkers, based on expression levels determined from sputum samples. The genes identified are *CLC*, *CPA3*, *DNASE1L3*, *IL1B*, *ALPL, IL8RB*, *HDC* and *PI3*. In particular the expression signature or profile of six genes (*CLC*, *CPA3*, *DNASE1L3*, *IL1B*, *ALPL* and *IL8RB)* is able to distinguish between asthma inflammatory phenotypes and predict response to corticosteroid treatment. Accordingly, provided herein are methods for determining the inflammatory phenotype (or distinguishing between inflammatory phenotypes) in asthma sufferers and methods for determining or predicting the responsiveness of a subject to asthma therapies, in particular corticosteroid therapy.

In broad terms, the present disclosure provides methods for distinguishing between asthma inflammatory phenotypes in a subject suffering from asthma, comprising determining the level of expression of one or more genes selected from *CLC*, *CPA3*, *DNASE1L3*, *IL1B*, *ALPL, IL8RB, HDC* and *PI3* in a biological sample isolated from the subject, wherein the expression profile of the one or more proteins in the sample is indicative of the inflammatory phenotype in the subject, wherein the inflammatory phenotype is selected from eosinophilic asthma, neutrophilic asthma and paucigranulocytic asthma. The inflammatory phenotypes may also be distinguished as eosinophilic versus non-eosinophilic asthma or as neutrophilic versus non-neutrophilic asthma or paucigranulocytic asthma versus non-paucigranulocytic asthma.

In a particular aspect the present disclosure provides a method for determining the asthma inflammatory phenotype in a subject suffering from asthma, the method comprising:
(a) isolating a biological sample from the subject; and
(b) determining the expression profile of the genes *CLC, CPA3*, *DNASE1L3, IL1B*, *ALPL* and *IL8RB,* wherein the expression profile comprises or is based on the expression levels of said genes in the sample,
wherein the expression profile of said genes is indicative of the asthma inflammatory phenotype.

Another aspect provides a method for distinguishing between eosinophilic asthma and non-eosinophilic asthma in a subject, the method comprising:
(a) isolating a biological sample from the subject; and
(b) determining the expression profile of the genes *CLC*, *CPA3*, *DNASE1L3*, *IL1B*, *ALPL* and *IL8RB,* based on the expression levels of said genes in the sample,
wherein the expression profile of said genes is indicative of the subject having eosinophilic asthma or non-eosinophilic asthma.

Methods disclosed herein enable the distinction between:
(i) eosinophilic asthma and non-eosinophilic asthma, typically where non-eosinophilic asthma is neutrophilic asthma or paucigranulocytic asthma;
(ii) neutrophilic asthma and non-neutrophilic asthma, typically where non-neutrophilic asthma is eosinophilic asthma or paucigranulocytic asthma;
(iii) neutrophilic asthma and paucigranulocytic asthma;
(iv) paucigranulocytic asthma and non-paucigranulocytic asthma, typically where non-paucigranulocytic asthma is neutrophilic asthma or eosinophilic asthma; or
(v) eosinophilic asthma and neutrophilic asthma.

Disclosed outside the scope of the claims are methods enabling:
(i) the distinction between eosinophilic asthma and non-eosinophilic asthma, eosinophilic asthma and paucigranulocytic asthma, or eosinophilic asthma or neutrophilic asthma, based on the expression levels of one or more genes selected from *CLC*, *CPA3*, *DNASE1L3* and *HDC*; and
(ii) the distinction between neutrophilic asthma and non-neutrophilic asthma, neutrophilic asthma and paucigranulocytic asthma, or neutrophilic asthma and eosinophilic asthma, based on the expression levels of one or more genes selected from *ALPL, IL1B, IL8RB* and *PI3.*

Typically, the correlation between expression of the protein(s) and an asthma inflammatory phenotype is determined by statistical analysis of the protein expression levels, such as by logistic regression analysis, as described herein.

It should be understood that reference to determining the level of expression of a gene is intended as a reference to the use of any suitable technique that will provide information in relation to the level of expression of the encoding nucleic acid molecule (DNA or mRNA) or the encoded protein or polypeptide in the relevant tissue of the subject. Accordingly, these techniques include both *in vivo* techniques, as well as *in vitro* techniques that are applied to a biological sample extracted from the subject. Such *in vitro* techniques are likely to be preferred due to their significantly more simplistic and routine nature. Those skilled in the art will readily appreciate that in accordance with embodiments of the invention gene expression may be determined by any suitable technique or assay known in the art. Methods disclosed herein typically require quantitation of expression levels. Gene expression may be measured, for example, using microarrays and/or using amplification based assays such as reverse-transcription PCR (RT-PCR) coupled with real time quantitative PCR (qPCR). At the protein or polypeptide level, expression may be determined, for example, by immunoassay using an antibody(ies) that bind with the protein such as enzyme-linked immunosorbent assay (ELISA) or immunoblotting, 2D-gel electrophoresis (including 2D-DIGE), multiplex protein expression assays, flow cytometry and protein expression profiling arrays and microarrays. The skilled addressee will appreciate that the invention is not limited by reference to the means by which gene expression is determined and/or quantified.

Methods of the present disclosure may be employed to determine or distinguish between asthma inflammatory phenotypes either in subjects that are known to have asthma (symptomatic or asymptomatic) or in subjects suspected of having asthma. Moreover, embodiments of the present disclosure may be used alone or in conjunction with, or as an adjunct to, one or more other diagnostic methods and tests to determine the asthma inflammatory phenotype experienced by a subject. Such other diagnostic methods and tests will be well known to those skilled in the art.

The expression profiles determined in samples from subjects in accordance with methods of the present disclosure can be compared to control values as a suitable reference to assist in diagnosis, for example such that abnormal expression profiles of genes in a sample from a subject of interest compared to the expression profiles of the same genes in one or more control samples is indicative of a specific inflammatory phenotype. For example, suitable control or reference expression profiles may be determined in one or more, typically a population, of individuals without an inflammatory phenotype of interest or known not to suffer from asthma. In subjects to which methods disclosed herein are applied, a comparison of the expression profiles with those obtained from the appropriate reference or control may determine diagnosis.

Reliable diagnoses of asthma inflammatory phenotypes, such as are possible utilising methods of the present disclosure also facilitate decision making with respect to the most appropriate intervention or treatment regime for individual subjects. The treatment regime may be tailored not only to the specific inflammatory phenotype suffered by the subject but also to the subject themselves based on one or more other factors such as the severity of the symptoms, the subjects lifestyle, age, weight, general health etc. For example, this may comprise introducing a new treatment regime or modifying an existing regime employed by the subject. The modification of a regime may be modification with respect to any one or more of a variety of factors, such as the nature of any anti-asthma medication, the dosage thereof, the timing of administration and/or any supplementary management strategies. Such decision making with respect to treatment regimes will vary from case to case and the determination of the most appropriate strategy is well within the expertise and experience of those skilled in the art.

A treatment regime for the treatment of asthma in a subject in accordance with the present disclosure may involve administration of any of the medications commonly utilised in the treatment of the disease. The treatment regime may comprise the administration of a number of drugs simultaneously, sequentially, or in combination with each other or with non-drug treatments. The type of drug(s) administered, dosage, and the frequency of administration can be determined by medical physicians in accordance with accepted medical principles, and will depend on factors such as the severity of the disease, the age and weight of the subject, the medical history of the subject, other medication being taken by the subject, existing ailments and any other health related factors normally considered when determining treatments for obstructive airways disease.

A commonly employed therapy in the treatment of asthma is corticosteroid inhalation. However treatment with corticosteroids is not effective in all individuals; whilst effectively treating eosinophilic asthma, corticosteroids are of little benefit in the treatment of individuals with non-eosinophilic inflammation. ICS response is greater in eosinophilic asthma compared to non-eosinophilic asthma. Recognition of eosinophilic asthma also results in better application of new therapies (Nair et al., 2009). Poor responses may be mediated by an inflammatory pattern that is insensitive to corticosteroids, thought to be the case for airway neutrophilia.

Accordingly, disclosed outside the scope of the claims methods for predicting whether a subject will respond to corticosteroid therapy, based on an analysis of the expression levels of one or more genes selected from *CLC, CPA3, DNASE1L3, IL1B* and *ALPL,* typically before and after administration of corticosteroids (for example by inhalation). In one embodiment the method comprises the steps of:
(a) obtaining a biological sample from the subject and determining the expression levels of the genes *CLC, CPA3*, *DNASE1L3, IL1B* and *ALPL* in the sample;
(b) administering a corticosteroid to the subject; and
(c) obtaining a further biological sample from the subject and determining the expression levels of the genes *CLC, CPA3, DNASE1L3*, *IL1B* and *ALPL* in the sample;
wherein a reduction in expression of one or more of *CLC, CPA3* and *DNASE1L3* and/or an increase in expression of one or more of *IL1B* and *ALPL* following administration step (b) is indicative that the subject is responsive to corticosteroid therapy.

In an alternative embodiment, the present disclosure provides a method for diagnosing responders to corticosteroid therapy for treating asthma, the method comprising the steps of:
(a) obtaining a biological sample from a subject;
(b) determining the expression profile of the genes *CLC, CPA3, DNASE1L3*, *IL1B*, *ALPL* and *IL8RB,* wherein the expression profile comprises or is based on the expression levels of said genes in the sample; and
(c) comparing the gene expression profile from the sample with a reference gene expression profile indicative of responsiveness to corticosteroid therapy, and/or to a reference gene expression profile indicative of non-responsiveness to corticosteroid therapy,
wherein similarity in expression profiles between the sample and reference profiles is indicative of the subject being a responder or non-responder to corticosteroid therapy.

The present disclosure also provides kits suitable for use in accordance with the methods of the disclosure. Such kits include for example diagnostic kits for assaying biological samples, comprising an agent(s) for detecting expression levels discriminatory proteins disclosed herein or encoding nucleic acid molecules, and reagents useful for facilitating the determination of expression by the agent(s). The agent(s) may be any suitable detecting molecule. Kits according to the present disclosure may also include other components required to conduct the methods of the present invention, such as buffers and/or diluents. The kits typically include containers for housing the various components and instructions for using the kit components in the methods of the present disclosure.

The reference in this specification to any prior publication (or information derived from it), or to any matter which is known, is not, and should not be taken as an acknowledgment or admission or any form of suggestion that that prior publication (or information derived from it) or known matter forms part of the common general knowledge in the field of endeavour to which this specification relates.

The present invention will now be described with reference to the following specific examples, which should not be construed as in any way limiting the scope of the invention.

### Examples

### General Methods

### Study Design

The inventors conducted 3 studies: a sputum gene expression profiling discovery study (n=47), a clinical validation study (n=80), and an evaluation of the biomarker signature to predict corticosteroid responsiveness (n=71). The discovery study assessed changes in the sputum gene expression profile of 47 subjects with stable non-smoking asthma that were related to asthma inflammatory phenotype (Table 1, eosinophilic asthma n=17; neutrophilic asthma n=12; paucigranulocytic asthma n=18). Gene biomarkers that were shown to be differentially expressed in the discovery microarray study were then validated by real-time PCR in this population. Those confirmed differentially regulated genes were followed up in a clinical validation study of 59 subjects with stable asthma (Table 2, eosinophilic asthma n=20; neutrophilic asthma n=18; paucigranulocytic asthma n=21) and a group of healthy volunteers (n=21). The top 6 genes were investigated for their ability to predict asthma inflammatory phenotype. Steroid response of 6 gene biomarkers was then assessed in a third population of asthmatics. The studies were approved by the relevant institutional ethics committees: The University of Newcastle Research Ethics Committee and the Lower South Regional Ethics Committee New Zealand.

### Study Population

For the discovery and clinical validation studies, adults with stable asthma were recruited from the John Hunter Hospital Ambulatory Care Clinic; NSW; Australia. For the corticosteroid responsiveness study, adults with stable persistent asthma were enrolled. Asthma was diagnosed according to American Thoracic Society guidelines based upon current (past 12 months) episodic respiratory symptoms, doctor's diagnosis (ever) and demonstrated evidence of airway hyperresponsiveness to hypertonic saline. Healthy nonasthmatic controls were recruited by advertisement. Exclusion criteria included: recent (past month) respiratory tract infection, recent asthma exacerbation, recent unstable asthma or change in maintenance therapy, and current smoking. Asthma inflammatory phenotype was assigned based on sputum eosinophil cut off of >3%, and sputum neutrophil cut off of >61% (Simpson *et al,* 2006).

### Sputum Induction and Analysis

Spirometry (KoKo PD Instrumentation, Louisville, USA) and sputum induction with hypertonic saline (4.5%) were performed as previously described (Simpson et al., 2006). For inflammatory cell counts, selected sputum was dispersed using dithiothreitol, and a total cell count viability was performed. Cytospins were prepared; stained (May-Grunwald Giemsa) and a differential cell count obtained from 400 non-squamous cells. For gene expression microarray analysis, 100µL of selected sputum was added to Buffer RLT (Qiagen, Hilden, Germany) and stored at -80°C until RNA extraction.

### Whole Genome Gene Expression Microarrays

RNA was extracted from induced sputum samples using the RNeasy Mini Kit (Qiagen, Hilden, Germany) and quantitated using the Quant-iT RiboGreen RNA Quantitation Assay Kit (Molecular Probes Inc, Invitrogen, Eugene, USA), as per manufacturers instructions. 500ng of RNA was reverse transcribed into cRNA and biotin-UTP labeled using the Illumina TotalPrep RNA Amplification Kit (Ambion, Texas, USA). 750ng of cRNA was hybridised to the Illumina Sentrix HumanRef-8 Version 2 Expression BeadChips, and scanned using the Illumina Bead Station and captured using BeadScan 3.5.11 (Illumina, San Diego, USA). Samples and gene profiling results were included in the analysis if the sample was of suitable purity (OD 260/280 1.7-2.1), successfully amplified (sufficient cRNA generated) and hybridized (GP95 score>500) and the data passed quality controls in GeneSpring GX (correlation coefficients and principle component analysis plots).

### Ranking of Differentially Expressed Genes as Potential Biomarkers

Differentially expressed genes due to asthma inflammatory phenotype were ranked in terms of 1) level of significance (ANOVA adjusted p value), 2) extent of the change expressed as fold difference between phenotypes, 3) abundance of mRNA expression in sputum 4) biological plausibility as to the candidates potential involvement in asthma pathogenesis; including their involvement in known biological pathways, and 5) importance and relevance as a biomarker and potential therapeutic target.

### Quantitative real-time PCR Validation

Sputum RNA (200ng) was reverse-transcribed to cDNA using the high capacity cDNA reverse transcription kit (Applied Biosystems, Foster City,USA). Taqman qPCR primer and probes were purchased in kit form (Applied Biosystems, Foster City,USA). PCR primers and probes were combined with Taqman gene expression master mix as per manufacturer's instructions in duplicate singleplex real-time PCRs (7500 Real Time PCR System, Applied Biosystems). Statistical analysis was performed on the change in cycle threshold (ΔCt) between the target gene and β-actin the housekeeping gene. Fold change results were calculated using 2^{-ΔΔCt} relative to both the housekeeping gene (β-actin) and the mean of the healthy control group.

### Statistical Analysis

Clinical and cell count data were analysed using Stata 9 (Stata Corporation, College Station, Texas,USA) and reported as mean (SD) for normally distributed data and median (Q1, Q3) for non-parametric data. Statistical comparisons were performed using the multiple comparison ANOVA for parametric data and the Kruskal Wallis test for non-parametric data, p<0.05 was considered significant.

Microarray data was exported using Genome Studio (Illumina, San Diego,USA) and analysed using GeneSpring GX11 (Agilent Technologies, Santa Clara,USA). Data were log transformed, normalised and baseline converted to the median of all samples. Data were filtered and only genes flagged as present (<0.05 detection p value) in about half of the samples (n=23/47, 49%) were included in the further analysis. Differential gene expression was determined using ANOVA, with Tukey post hoc testing (p<0.05 adjusted for multiple comparisons using Benjamini Hochberg method) and fold change of >2.

Logistic regression was used to calculate the predicted value of an individual having the disease phenotype based on their level of a particular marker (simple logistic regression) or combination of markers (multiple logistic regression). Receiver Operating Characteristic (ROC) Curves were generated from the predicted values of the regression analyses, and the predicted values, the detection rate and the area under the curve (AUC) were calculated. Bland-Altman plots and Intra Class Correlation (ICC) coefficients were used to determine reproducibility.

### Example 1 - Differentially Expressed Genes between Asthma Inflammatory Phenotypes

Whole genome gene expression profiles (including 22,184 entities) were generated from induced sputum samples from 17 subjects with eosinophilic asthma, 12 subjects with neutrophilic asthma and 18 subjects with paucigranulocytic asthma (Table 1).

A gene was considered to be expressed if it was flagged as present in about half (n=23/47, 49%) of samples tested, and this resulted in 14,573 entities out of 22,184 (65.7%) being expressed. Out of these 14,573 entities, 5284 were significantly different due to asthma inflammatory phenotype (ANOVA, p<0.05 adjusted for multiple comparisons using Benjamini Hochberg false discovery rate). There were 277 entities that had a >2 change of gene expression between comparisons (data not shown), of which 36 differentially expressed genes were selected as candidates for validation by real-time PCR.

The real-time PCR results for all genes analysed were significantly correlated with the array results, however, only 28 genes remained significantly differentially expressed between asthma inflammatory phenotypes (Table 2), with 9 of these genes reaching a significance of p≤0.001. Expression of *BIRC3*, *CCR7, CPA3, CEBPE*, *CLC, DNASE1L3*, *HDC,* and *TFF3* were significantly highest in eosinophilic asthma. Expression of *ALPL, CXCR1*, *CXCR2*, *IL1B*, *IL1R2*, *IL1RN*, *IRAK2*, *ISG20*, *NFKB2*, *NFKBIZ, NLRP3, ORM1*, *PELI1*, *PI3, TNFAIP3*, *TNFRSF1B* and *VNN2* were highest in neutrophilic asthma. Expression of *DEFB1* was highest in paucigranulocytic astma, and *TNFSF14* was similarly increased in both eosinophilic asthma and neutrophilic asthma compared to paucigranulocytic asthma. *CAMP*, *FPR2*, *GBP1*, *GCHFR, IL18RAP, NAMPT* and *PLAU* although correlated with the array results, did not reach significance between inflammatory phenotypes, so were not tested in the clinical validation population.

**Table 1: Clinical characteristics of the participants in the microarray discovery study**

| | **Eosinophilic** | **Neutrophilic** | **Paucigranulocytic** | **p** |
|---|---|---|---|---|
| **N** | 17 | 12 | 18 | |
| **Age years, mean (SD)** | 59 (18) | 59 (11) | 56 (11) | 0.811 |
| **Gender M** \| **F** | 7 \| 10 | 6 \| 6 | 7 \| 11 | 0.825 |
| **Atopy, n (%)** | 16 (94)*^{∥} | 6 (50) | 9 (50) | 0.004 |
| **FEV₁% predicted, mean (SD)** | 67 (15)* | 71 (23) | 87 (18) | 0.005 |
| **FEV₁/FVC %, mean (SD)** | 62 (10)* | 66 (12) | 74 (6) | 0.002 |
| **FENO (ppb), median (Q1, Q3)** | 52.4 (36.2, 76.2)^{†§} | 22.8 (14.1, 27.0) | 16.3 (11.2, 21.3) | <0.001 |
| **Smoking, Ex\| Never** | 9 \| 8 | 5 \| 7 | 6 \| 12 | 0.502 |
| **Pack years, median (Q1, Q3)** | 10.0 (0.5, 17.0) | 5.0 (5.0, 7.5) | 4.4 (0.7, 24.0) | 0.897 |
| **Asthma Control Score, median (Q1, Q3)** | 1.3 (0.9, 1.7)^{†} | 1.0 (0.8, 2.1)^{†} | 0.5 (0.3, 0.7) | 0.005 |
| **Inhaled Corticosteroid (ICS) Use, Yes\| No** | 11 \| 6 | 10 \| 2 | 10 \| 8 | 0.291 |
| **ICS dose (µg daily BDP equivalent) median (Q1, Q3)** | 800 (400, 1000) | 1000 (200, 2000) | 750 (400, 2000) | 0.738 |
| **Total cell count x 10⁶/mL, median (Q1, Q3)** | 3.2 (2.4, 5.5) | 5.0 (2.9, 8.6)^{†} | 2.5 (1.4, 3.4) | 0.044 |
| **Viability, median (Q1, Q3)** | 69.2 (56.6, 88.5) | 90.2 (78.2, 95.3)^{‡} | 81.7 (71.9, 90.8) | 0.032 |
| **Neutrophils, %, median (Q1, Q3)** | 23.5 (14.8, 43.3) | 78.1 (75.4, 83.3)^{†‡} | 36.2 (21.0, 43.8) | <0.001 |
| **Eosinophils, % median (Q1, Q3)** | 23.8 (13.5, 46.3)^{†§} | 0.6 (0.2, 0.8) | 0.0 (0.0, 0.5) | <0.001 |
| **Macrophages, %, median (Q1, Q3)** | 36.8 (26.8, 41.8) | 18.9 (15.9, 22.4) | 62.3 (55.0, 75.3)^{§‡} | <0.001 |
| **Lymphocytes, %, median (Q1, Q3)** | 0.3 (0.3, 1.3) | 0.1 (0.0, 0.6) | 0.8 (0.1, 2.0) | 0.060 |
| **Columnar epithelial, %, median (Q1, Q3)** | 1.9 (0.0, 2.5) | 0.8 (0.3, 3.6) | 1.0 (0.0, 4.6) | 0.936 |
| **Squamous, %, median (Q1, Q3)** | 3.4 (1.5, 8.1) | 2.6 (0.6, 9.3) | 3.8 (2.2, 6.4) | 0.965 |

| | | | | |
|---|---|---|---|---|
| Bonferroni post hoc test *p<0.017 versus PGA; kwallis2 ^{†}p<0.008 versus PGA; ^{‡}p<0.008 versus EA; ^{§}p<0.008 versus NA; ^{∥}Chi squared test p<0.017 | | | | |

**Table 2: Differential expression of biomarker candidates in both study groups determined by qPCR^**

| **Gene Symbol** | | **Discovery Asthma Population** | | | | **Validation Asthma Population** | | | |
|---|---|---|---|---|---|---|---|---|---|
| | | **Eosinophilic** | **Neutrophilic** | **Paucigranulocytic** | **p** | **Eosinophilic** | **Neutrophilic** | **Paucigranulocytic** | **p** |
| | n | 17 | 12 | 16 | | 20 | 18 | 21 | |
| | | | | | | | | | |
| 1 | ALPL | 5.82 (1.92) | 4.28 (2.32)* | 7.64 (2.39) | 0.001 | 5.67 (1.72) | 3.42 (1.63)*^{†} | 5.73 (1.75) | <0.001 |
| 2 | BIRC3 | 5.42 (2.46)* | 5.52 (1.83) | 7.70 (1.56) | 0.004 | 4.71 (1.85) | 4.71 (1.74) | 5.86 (1.18) | 0.034 |
| 3 | CAMP | 7.93 (1.80) | 6.85 (1.63) | 7.10 (1.56) | 0.189 | | | | |
| 4 | CCR7 | 5.56 (1.68)* | 6.54 (1.79) | 8.04 (2.04) | 0.002 | 6.45 (2.18) | 6.04 (2.09) | 7.33 (2.39) | 0.209 |
| 5 | CEBPE | 8.21 (2.41)* | 10.36 (1.92)* | 12.75 (1.44) | <0.001 | 10.01 (2.40) | 10.71 (1.77) | 11.56 (1.84) | 0.052 |
| 6 | CLC | 6.24 (3.01)*^{#} | 9.02 (2.10)* | 11.97 (2.02) | <0.001 | 6.99 (3.75)* | 9.21 (2.75) | 10.55 (1.74) | <0.001 |
| 7 | CPA3 | 7.89 (1.96)*^{#} | 11.12 (2.54) | 12.79 (2.84) | <0.001 | 8.55 (2.07)*^{#} | 10.90 (2.39) | 11.81 (2.00) | <0.001 |
| 8 | DEFB1 | 8.14 (1.62)* | 7.76 (2.19) | 6.29 (1.29) | 0.008 | 6.95 (2.55) | 8.65 (2.37) | 7.41 (1.25) | 0.046 |
| 9 | DNASE1L3 | 10.06 (2.68)* | 11.32 (1.61)* | 14.01 (2.27) | <0.001 | 9.98 (2.24)* | 11.37 (1.76) | 12.18 (2.30) | 0.005 |
| 10 | FPR2 | 6.38 (2.09) | 6.00 (2.68) | 7.64 (2.07) | 0.131 | | | | |
| 11 | GBP1 | 5.08 (1.54) | 4.18 (1.74) | 5.28 (1.53) | 0.183 | | | | |
| 12 | GCHFR | 3.78 (1.36) | 3.93 (1.74) | 3.10 (0.51) | 0.173 | | | | |
| 13 | HDC | 10.75 (2.58)* | 12.58 (2.14) | 14.82 (1.59) | <0.001 | 11.89 (1.60)* | 12.59 (1.88) | 14.23 (2.46) | 0.002 |
| 14 | IL18RAP | 8.34 (1.52) | 7.54 (1.31) | 9.49 (2.85) | 0.051 | 7.86 (1.95) | 6.51 (1.73)* | 8.39 (1.78) | 0.006 |
| 15 | IL1B | 3.68 (2.38) | 1.87 (2.38)* | 5.04 (1.67) | 0.002 | 2.47 (1.75) | 0.78 (1.78)*^{†} | 2.82 (1.26) | <0.001 |
| 16 | IL1R2 | 5.97 (1.74)* | 5.05 (1.71)* | 7.82 (1.99) | <0.001 | 6.00 (2.19) | 3.98 (1.68)^{*†} | 6.26 (1.78) | <0.001 |
| 17 | IL1RN | 4.80 (2.08) | 4.05 (2.04)* | 6.82 (2.64) | 0.006 | 4.16 (1.32) | 3.04 (1.83)* | 4.57 (1.04) | 0.004 |
| 18 | IL8RA (CXCR1) | 9.30 (2.29) | 8.12 (2.69) | 10.38 (2.07) | 0.050 | 7.92 (1.49) | 6.57 (1.23)*^{†} | 8.82 (1.18) | <0.001 |
| 19 | IL8RB (CXCR2) | 5.52 (1.35) | 4.41 (1.68)* | 6.45 (1.38) | 0.003 | 4.08 (1.71) | 2.45 (2.01)*^{†} | 4.66 (1.39) | <0.001 |
| 20 | IRAK2 | 4.24 (1.64) | 3.42 (3.58)* | 6.04 (1.80) | 0.015 | 4.55 (1.57) | 2.37 (2.36)*^{†} | 4.58 (1.72) | <0.001 |
| 21 | ISG20 | 4.55 (1.70) | 3.60 (1.51)* | 5.84 (1.98) | 0.006 | 5.51 (1.55) | 3.55 (1.68)*^{†} | 5.66 (1.57) | <0.001 |
| 22 | NAMPT | 3.81 (2.60) | 3.28 (1.76) | 4.14 (1.76) | 0.570 | | | | |
| 23 | NFKB2 | 4.35 (1.63) | 3.92 (1.89)* | 6.08 (1.73) | 0.004 | 4.92 (1.00) | 3.23 (1.64)*^{†} | 4.99 (1.33) | <0.001 |
| 24 | NFKBIZ | 4.23 (1.52) | 3.75 (1.88)* | 5.73 (1.57) | 0.006 | 4.07 (1.10) | 2.62 (1.84)*^{†} | 4.68 (1.14) | <0.001 |
| 25 | NLRP3 | 5.60 (1.79)* | 5.61 (2.19)* | 8.03 (1.70) | <0.001 | 5.78 (1.62) | 4.43 (2.13)* | 6.43 (1.97) | 0.007 |
| 26 | ORM1 | 10.08 (3.15) | 8.34 (3.29)* | 12.23 (2.85) | 0.007 | 8.76 (2.88) | 6.30 (3.16)* | 10.16 (2.78) | <0.001 |
| 27 | PELI1 | 5.17 (1.92) | 4.04 (2.03)* | 6.55 (1.61) | 0.004 | 4.46 (1.92) | 3.65 (1.53)* | 5.17 (1.24) | 0.013 |
| 28 | PI3 | 4.95 (3.34) | 2.84 (2.91)* | 7.17 (2.66) | 0.002 | 4.68 (2.89) | 2.02 (2.54)*^{†} | 4.50 (2.36) | 0.005 |
| 29 | PLAU | 4.34 (2.39) | 3.85 (1.66) | 4.96 (1.73) | 0.349 | | | | |
| 30 | TFF3 | 4.62 (2.18)*^{#} | 7.06 (1.06) | 7.41 (2.57) | <0.001 | 5.47 (2.22) | 7.16 (2.52) | 6.94 (1.80) | 0.034 |
| 31 | TNFAIP3 | 3.22 (1.72) | 2.43 (1.97)* | 4.68 (1.66) | 0.005 | 2.98 (1.47) | 1.83 (2.16)* | 3.39 (1.32) | 0.016 |
| 32 | TNFRSF1B | 4.04 (1.76) | 3.48 (2.05)* | 5.73 (1.65) | 0.004 | 4.71 (1.10) | 3.12 (1.51)*^{†} | 4.92 (1.23) | <0.001 |
| 33 | TNFSF14 | 5.58 (1.85)* | 5.46 (2.03)* | 7.59 (1.77) | 0.004 | 6.33 (1.60) | 3.85 (2.53)*^{†} | 6.43 (2.13) | <0.001 |
| 34 | VNN2 | 6.19 (2.20) | 4.75 (2.53)* | 7.82 (2.60) | 0.007 | 4.73 (1.70) | 3.19 (1.48)*^{†} | 5.13 (1.00) | <0.001 |

| | | | | | | | | | |
|---|---|---|---|---|---|---|---|---|---|
| ^Data expressed as the change in cycle threshold (Ct) compared to the housekeeping gene β-actin (ΔCt), mean (SD). A lower ΔCt corresponds to a stronger expression of the target gene. *p<0.017 bonferroni post hoc test versus paucigranulocytic asthma, ^{#}p<0.017 bonferroni post hoc test versus neutrophilic asthma, ^{†} p<0.017 bonferroni post hoc test versus eosinophilic asthma | | | | | | | | | |

### Example 2 - Validation of Candidate Genes in the Secondary Validation Asthma Population

The inventors then tested the 28 validated candidate gene expression biomarkers in a secondary population of subjects with eosinophilic asthma n=20, neutrophilic asthma n=18, and paucigranulocytic asthma n=21 (Table 3). There were 26 genes that remained significantly different between inflammatory phenotypes of asthma (see Table 2), with 15 of these reaching a significance of p≤0.001. Expression of *BPIFB1*, *CLC*, *CPA3*, *DNASE1L3*, and *HDC* were the highest in eosinophilic asthma, whereas expression of *ALPL,* CXCR1, *CXCR2, IL1B*, *IL1R2, IL1RN, IRAK2, ISG20, NFKB2, NFKBIZ,* NLRP3, *ORM1, PELI1, PI3, TNFAIP3, TNFRSF1B, TNFSF14* and *VNN2* were highest in neutrophilic asthma. Although expression of *BIRC3, DEFB1,* and *TFF3* reached significance overall, a post hoc analysis of individual phenotypes was not significant. *CCR7* and *CEBPE* did not reach significance in the clinical validation population. This left 23 genes consistently different between inflammatory phenotype in both discovery and validation populations.

### Example 3 - Analysis of the Biomarker Signature

The performance of the selected gene expression biomarkers to discriminate asthma inflammatory phenotype was investigated as single markers (using simple logistic regression) and as a combination of biomarkers (using multiple logistic regression). Only those genes that were confirmed using real-time PCR as significantly different between phenotype in both the discovery and the validation population were further investigated for their potential to predict asthma inflammatory phenotype. 4 genes (*CLC*, *CPA3*, *DNASE1L3* and *HDC)* were further investigated for their potential to predict eosinophilic asthma. Each of these markers individually was able to discriminate eosinophilic asthma from non-eosinophilic asthma, as well as neutrophilic and paucigranulocytic asthma separately (Table 4). *CPA3* was the best individual discriminator for eosinophilic asthma, followed by *CLC*, *DNASE1L3* and *HDC.*

**Table 3: Clinical characteristics of the participants in the clinical validation population**

| | **Healthy controls** | **Eosinophilic** | **Neutrophilic** | **Paucigranulocytic** | **p** |
|---|---|---|---|---|---|
| **N (% total)** | 20 | 20 | 18 | 21 | |
| **Age years, mean (SD)** | 43 (19) | 54 (17) | 59 (12)* | 60 (13)* | 0.003 |
| **Gender** M F | 8 \| 12 | 8 \| 12 | 8 \| 10 | 13 \| 8 | 0.300 |
| **Atopy, n (%)** | 8 (40) | 13 (65) | 10 (56) | 15 (71) | 0.198 |
| **FEV₁% predicted, mean (SD)** | 99 (12) | 73 (17)* | 71 (23)* | 87 (22) | <0.001 |
| **FEV₁/FVC %, mean (SD)** | 79 (7) | 65 (10)* | 65 (11)* | 69 (7)* | <0.001 |
| **FENO (ppb), median (Q1, Q3)** | 16.84 (14.7, 236) | 49.5 (20.5, 71.0)^{†§‡} | 18.6 (12.6, 26.2) | 17.0 (14.1, 27.6) | 0.004 |
| **Smoking, Ex\| Never** | 6 \| 14 | 13 \| 7 | 7 \| 11 | 11 \| 10 | 0.213 |
| **Pack years, median (Q1, Q3)** | 19.5 (29, 38.8) | 0.6 (0.4, 0.7)^{†§} | 24.0 (3.6, 32.0)^{‡} | 10.0 (2.0, 25.0) | 0.004 |
| **Asthma Control Score, median (Q1, Q3)** | N/A | 1.1 (0.8, 1.7)^{‡} | 1.1 (0.8, 1.5)^{‡} | 0.4 (0.3, 0.8) | 0.005 |
| **Inhaled Corticosteroid (ICS) Use, No \|Yes** | N/A | 3 \| 17 | 3 \| 15 | 3 \| 18 | 0.978 |
| **ICS dose (µg daily BDP equivalent) median (Q1, Q3)** | N/A | 1000 (500, 2000) | 2000 (800, 2000) | 1000 (1000, 2000) | 0.523 |
| **Total cell count x 10⁶/mL, median (Q1,Q3)** | 3.9 (1.9, 5.4) | 4.4 (2.7, 4.8) | 10.8(6.7, 15.5)^{†‡∥} | 2.6 (1.8, 4.1) | <0.001 |
| **Viability, median (Q1,Q3)** | 75.9 (67.6, 85.0) | 74.6 (64.6, 84.6) | 91.4 (87.1, 94.6)^{†‡∥} | 67.3 (56.9, 75.9) | <0.001 |
| **Neutrophils, %, median** | 28.4 (12.1, 50.34) | 27.4 (18.3, 38.8) | 76.1 (71.5, 87.0)^{†‡∥} | 26.3 (17.0, 48.8) | <0.001 |
| **(Q1,Q3)** | | | | | |
| **Eosinophils, % median (Q1,Q3)** | 0.3 (0.1, 0.8) | 11.9 (4.4, 20.6)^{†§‡} | 0.8 (0.0, 1.5) | 0.3 (0.0, 0.5) | <0.001 |
| **Macrophages, %, median (Q1,Q3)** | 68.0 (41.6, 74.0) | 48.8 (40.3, 61.0) | 20.8 (11.8, 24.3)^{†‡∥} | 67.0 (50.3, 76.5) | <0.001 |
| **Lymphocytes, %, median (Q1, Q3)** | 1.3 (0.4, 3.1) | 0.3 (0.3, 2.0) | 0.5 (0.0, 0.8) | 0.3 (0.0, 1.0)^{†} | 0.035 |
| **Columnar epithelial, %, median (Q1, Q3)** | 1.9 (0.6, 6.4) | 1.5 (0.8, 2.9) | 0.6 (0.0, 1.8) | 2.0 (0.8, 4.0) | 0.112 |
| **Squamous, %, median (Q1, Q3)** | 4.2 (0.8, 6.5) | 4.1 (2.0, 6.1) | 1.5 (0.7, 2.7) | 2.9 (2.0, 9.3) | 0.215 |

| | | | | | |
|---|---|---|---|---|---|
| Bonferroni post hoc test *p<0.017 versus PGA; kwallis2 ^{†}p<0.01 versus healthy controls; ^{‡}p<0.01 versus paucigranulocytic asthma; ^{‡}p<0.01 versus neutrophilic asthma; ^{∥}p<0.01 versus eosinophilic asthma; BDP = beclomethasone dipropionate | | | | | |

**Table 4: Analysis of diagnostic value of 4 individual gene expression biomarkers to diagnose eosinophilic asthma**

| | | | | **Minimal False Negatives** | | | **Minimal False Positives** | | |
|---|---|---|---|---|---|---|---|---|---|
| **Comparison** | **Gene Biomarker** | **AUC %** | **P value** | Cutoff Predicted Value | Sensitivity | Specificity | Cutoff Predicted Value | Sensitivity | Specificity |
| EA vs. NEA | CLC | 80.2 | <0.0001 | ≥0.319 | 78.4% | 74.6% | ≥0.411 | 73.0% | 83.6% |
| | CPA3 | 86.1 | <0.0001 | ≥0.256 | 89.2% | 67.2% | ≥0.407 | 70.3% | 88.1% |
| | DNASE1L3 | 76.5 | <0.0001 | ≥0.351 | 75.7% | 65.7% | ≥0.422 | 67.6% | 77.6% |
| | HDC | 75.5 | <0.0001 | ≥0.346 | 70.3% | 65.2% | ≥0.419 | 56.8% | 72.7% |
| EA vs. PGA | CLC | 86.6 | <0.0001 | ≥0.308 | 86.5% | 67.6% | ≥0.667 | 73.0% | 91.9% |
| | CPA3 | 90.6 | <0.0001 | ≥0.294 | 94.6% | 62.2% | ≥0.634 | 70.3% | 94.6% |
| | DNASE1L3 | 80.3 | <0.0001 | ≥0.440 | 86.5% | 70.3% | ≥0.534 | 75.7% | 78.4% |
| | HDC | 84.7 | <0.0001 | ≥0.338 | 91.9% | 63.9% | ≥0.569 | 70.3% | 77.8% |
| EA vs. NA | CLC | 72.3 | 0.002 | ≥0.496 | 75.7% | 66.7% | ≥0.544 | 73.0% | 73.3% |
| | CPA3 | 80.5 | <0.0001 | ≥0.438 | 86.5% | 63.3% | ≥0.553 | 73.0% | 80.0% |
| | DNASE1L3 | 71.9 | 0.006 | ≥0.569 | 70.3% | 73.3% | ≥0.572 | 67.6% | 76.7% |
| | HDC | 64.4 | 0.019 | ≥0.511 | 67.6% | 53.3% | ≥0.567 | 51.4% | 60.0% |

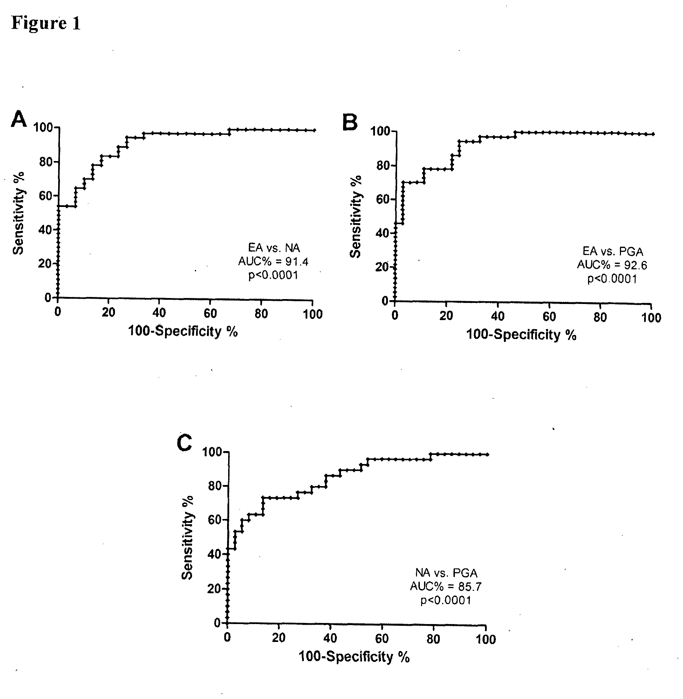

The 4 most significant (ANOVA p<0.003 for both the discovery and validation populations) genes (*ALPL, IL1B, IL8RB* and *PI3*) were further investigated for their potential to predict neutrophilic asthma. Each of these markers individually was able to discriminate neutrophilic asthma from non-neutrophilic asthma, as well as paucigranulocytic and eosinophilic asthma separately (Table 5). *IL1B* was the best individual discriminator for neutrophilic asthma, followed by *ALPL*, *IL8RB* and *PI3.* 2, 3, 4, 5 and 6 multiple combinations of the top 6 gene biomarkers were then evaluated across all phenotype comparisons. The combination of 6 genes including *CLC, CPA3*, *DNASE1L3*, *IL1B*, *ALPL*, *IL8RB* gave the strongest performance to discriminate asthma inflammatory phenotypes (Table 6). The 6 gene signature was able to discriminate eosinophilic asthma from non-eosinophilic asthma (AUC 89.6%, p<0.0001), neutrophilic asthma (Figure 1A, AUC 91.4%, p<0.0001), and paucigranulocytic asthma (Figure 1B, AUC 92.6%, p<0.0001). The 6 gene signature was also able to discriminate neutrophilic asthma from non-neutrophilic asthma (AUC 84.5%, p<0.0001), and paucigranulocytic asthma (Figure 1C, AUC 85.7%, p<0.0001).

**Table 5: Analysis of diagnostic value of 4 individual gene expression biomarkers to diagnose neutrophilic asthma**

| | | | | **Minimal False Negatives** | | | **Minimal False Positives** | | |
|---|---|---|---|---|---|---|---|---|---|
| **Comparison** | **Gene Biomarker** | **AUC %** | **P value** | Cutoff Predicted Value | Sensitivity | Specificity | Cutoff Predicted Value | Sensitivity | Specificity |
| NA vs. NNA | ALPL | 71.9 | <0.0001 | ≥0.234 | 86.7% | 64.9% | ≥0.397 | 63.3% | 79.7% |
| | IL1B | 78.9 | <0.0001 | ≥0.248 | 73.3% | 64.9% | ≥0.347 | 63.3% | 77.0% |
| | IL8RB | 77.6 | <0.0001 | ≥0.269 | 76.7% | 67.6% | ≥0.369 | 66.7% | 82.4% |
| | PI3 | 75.8 | <0.0001 | ≥0.250 | 73.3% | 59.5% | ≥0.351 | 56.7% | 78.4% |
| NA vs. PGA | ALPL | 81.0 | <0.0001 | ≥0.438 | 83.3% | 73.0% | ≥0.536 | 70.0% | 78.4% |
| | IL1B | 83.1 | <0.0001 | ≥0.337 | 83.3% | 64.9% | ≥0.519 | 70.0% | 78.4% |
| | IL8RB | 81.9 | <0.0001 | ≥0.360 | 83.3% | 62.2% | ≥0.519 | 70.0% | 81.1% |
| | PI3 | 78.8 | <0.0001 | ≥0.355 | 83.3% | 59.5% | ≥0.560 | 56.7% | 86.5% |
| NA vs. EA | ALPL | 77.4 | <0.0001 | ≥0.495 | 75.7% | 63.3% | ≥0.648 | 62.2% | 86.7% |
| | IL1B | 74.8 | 0.0006 | ≥0.529 | 73.0% | 63.3% | ≥0.632 | 62.2% | 76.7% |
| | IL8RB | 73.2 | 0.0008 | ≥0.512 | 81.1% | 66.7% | ≥0.597 | 62.2% | 76.7% |
| | PI3 | 72.7 | 0.0009 | ≥0.539 | 70.3% | 56.7% | ≥0.612 | 56.8% | 73.3% |

**Table 6: Analysis of diagnostic values of CLC, CPA3, DNASE1L3, ALPL, IL1B, IL8RB in combination for asthma inflammatory phenotype**

| | | | **Minimal False Negatives** | | | **Minimal False Positives** | | |
|---|---|---|---|---|---|---|---|---|
| **Comparison** | **AUC%** | **P value** | Cutoff Predicted Value | Sensitivity | Specificity | Cutoff Predicted Value | Sensitivity | Specificity |
| | | | | | | | | |
| EA vs. NEA | 89.6 | <0.0001 | ≥0.250 | 89.2% | 73.1% | ≥0.519 | 70.3% | 91.0% |
| EA vs. PGA | 92.6 | <0.0001 | ≥0.361 | 94.6% | 75.7% | ≥0.782 | 70.3% | 97.3% |
| EA vs. NA | 91.4 | <0.0001 | ≥0.372 | 94.6% | 73.3% | ≥0.653 | 78.4% | 86.7% |
| NA vs. NNA | 84.5 | <0.0001 | ≥0.290 | 86.7% | 75.7% | ≥0.398 | 73.3% | 82.4% |
| NA vs. PGA | 85.7 | <0.0001 | ≥0.330 | 86.7% | 62.2% | ≥0.543 | 73.3% | 86.5% |
| PGA vs. NPGA | 85.4 | <0.0001 | ≥0.244 | 91.9% | 67.2% | ≥0.458 | 70.3% | 83.6% |

### Example 4 - Validation of 6 Gene Expression Biomarker Signature compared to Healthy Controls

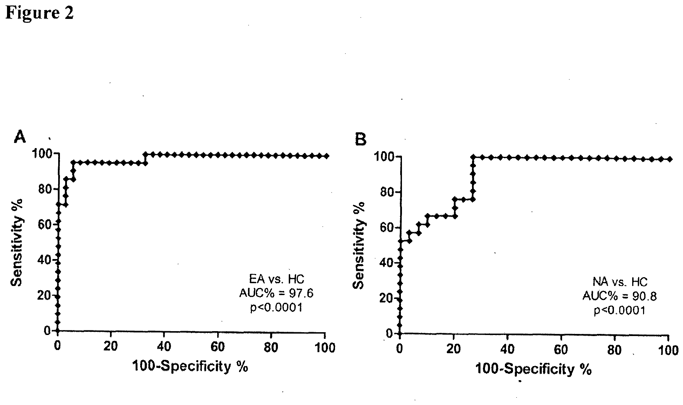

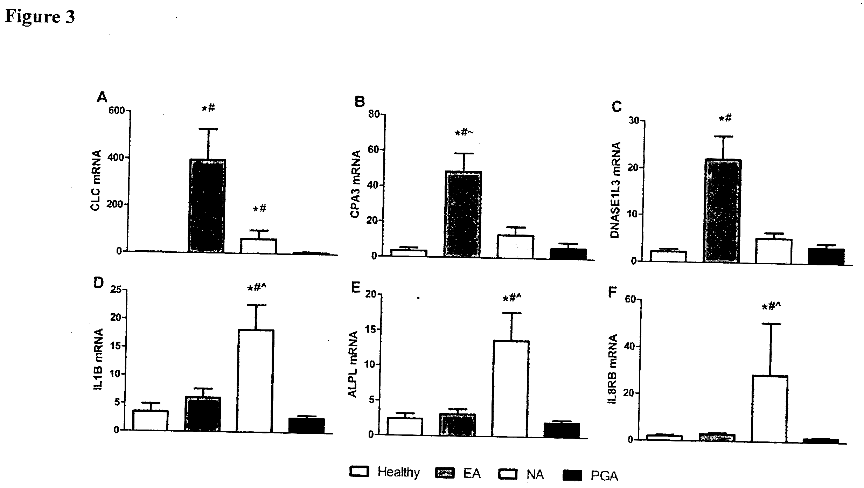

The expression of the 6 gene expression biomarkers (*CLC*, *CPA3*, *DNASE1L3*, *IL1B*, *ALPL* and *IL8RB*) was further investigated in comparison to induced sputum samples from healthy control subjects. The 6 gene expression biomarker signature was able to discriminate asthma from healthy controls with 85% accuracy, eosinophilic asthma from healthy controls with 98% accuracy (Figure 2A), and neutrophilic asthma from healthy controls with 91% accuracy (Figure 2B). As expected, *CLC* (Figure 3A), *CPA3* (Figure 3B) and *DNASE1L3* (Figure 3C) had significantly increased gene expression in eosinophilic asthma compared to healthy controls. *IL1B* (Figure 3D), *ALPL* (Figure 3E) and *IL8RB* (Figure 3F) had significantly increased gene expression in neutrophilic asthma compared to healthy controls.

Sputum gene expression of the 6 biomarkers was measured in 30 subjects (n=9 EA, n=7 NA, n=14 PGA) on 2 occasions, an average of 30 days apart. The bias of measurement was small with equal scatter for all genes (data not shown). ICC coefficients were almost perfect for *CLC* (0.84), substantial for *CPA3* (0.71), *DNASE1L3* (0.75), and *IL1B* (0.66), and moderate for *ALPL* (0.55) and *CXCR2* (0.47).

### Example 5 - Assessment of the 6 Gene Biomarker Signature to predict Steroid Responsiveness

To investigate the responsiveness of the 6 gene biomarker signature to inhaled corticosteroids (ICS) the expression of the markers were investigated in 71 asthmatics before and after corticosteroid treatment (1000µg fluticasone per day for 28 days). The effects of ICS treatment on inflammatory phenotype of asthma and other information regarding this population have been previously published (Cowan *et al,* 2010), and consisted of subjects with eosinophilic (n=48) and non-eosinophilic (n=23) asthma. Neutrophilic asthma was rare in this population. The 6 gene biomarker signature successfully predicted 92% of subjects with eosinophilic asthma, and 74% of subjects with non-eosinophilic asthma (Table 7).

**Table 8: Predicted Phenotypes in New Zealand Population**

| | | **ACTUAL PHENOTYPE** | | **TOTAL** |
|---|---|---|---|---|
| | | EA | NEA | |
| **PREDICTED PHENOTYPE** | **EA'** | 44 | 6 | 50 |
| | **NEA'** | 4 | 17 | 21 |
| | **TOTAL** | 48 | 23 | |
| **% Accuracy** | | 92% | 74% | |

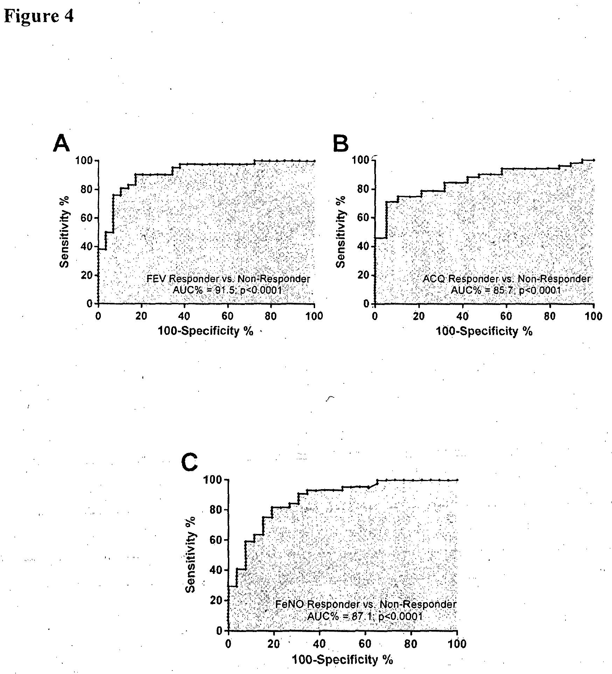

Comparison of the sputum cell counts and the gene signature methods to determine inflammatory phenotype showed an 86% agreement with a κ statistic of 0.67 (p<0.0001), which is a substantial agreement according to the Landis and Koch classifications. Further statistical testing demonstrated that the 6 gene biomarker signature could discriminate ICS responsiveness particularly a >12% improvement in FEV1 (Figure 4A; AUC 91.5%; p<0.0001), 0.5 point decrease in ACQ score (Figure 4B; AUC=85.7%; p=0.0022), or a 40% decrease in FENO (Figure 4C, AUC=87.1%; p<0.0001)

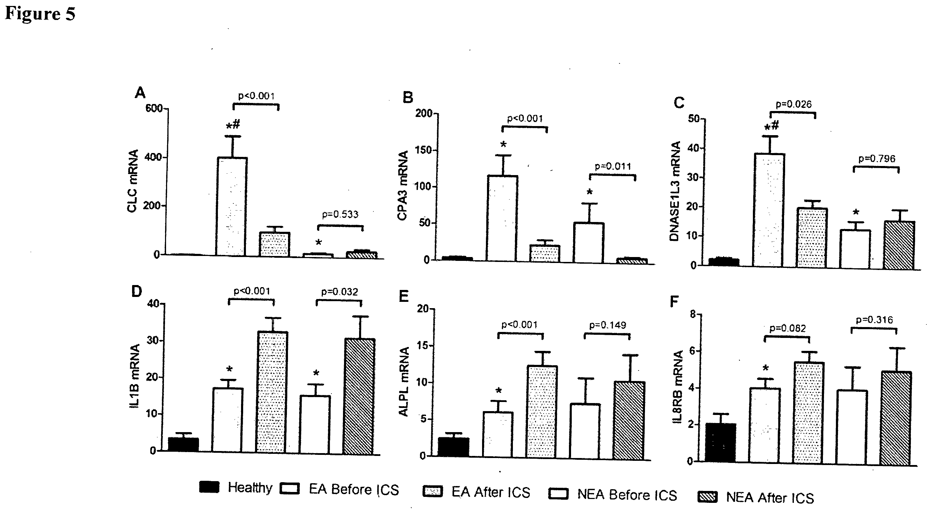

All 6 gene expression biomarkers were significantly elevated in eosinophilic asthma compared to healthy controls (Figure 5). Expression of *CLC* (Figure 5A), and *DNASE1L3* (Figure 5C) were also significantly elevated in eosinophilic asthma compared to non-eosinophilic asthma. Expression of IL1B was significantly higher in both eosinophilic and non-eosinophilic asthma compared to healthy controls (Figure 5D). In response to steroid treatment, the expression of *CLC* (Figure 5A), *CPA3* (Figure 5B) and *DNASE1L3* (Figure 5C) were reduced in eosinophilic asthma, and *CPA3* was also reduced in non-eosinophilic asthma. In contrast, the expression of *IL1B* (Figure 5D) and *ALPL* (Figure 5E) were increased with steroid treatment of eosinophilic asthma.

### Discussion

The study described in the above examples identified a 6 gene expression biomarker signature derived from induced sputum samples that reproducibly discriminates the inflammatory phenotypes of asthma and predicts ICS responsiveness. The top 6 genes including CLC, CPA3, DNASE1L3, IL1B, ALPL and CXCR2 in combination were shown to accurately identify asthma inflammatory phenotype, thereby demonstrating their potential use as a clinical biomarker. CLC, CPA3 and DNASE1L3 are shown to have increased gene expression in eosinophilic asthma, whereas IL1B, ALPL and CXCR2 have increased gene expression in neutrophilic asthma. This 6 gene expression signature distinguishes eosinophilic asthma from non-eosinophilic asthma with substantial agreement, and could predict ICS responsiveness defined by a greater than 12% decrease in FEV1, 0.5 decrease in ACQ score and 40% reduction in FENO. The 6 gene biomarker signature could predict asthma and phenotype of asthma from healthy controls and demonstrated predominant eosinophilia and neutrophilia in MGA. Expression levels of the 6 gene signature were modified by ICS treatment, with CLC, CPA3 and DNASEIL3 being decreased and IL1B and ALPL being increased after ICS treatment of eosinophilic asthma.

### References

Cowan DC, et al. Effects of steroid therapy on inflammatory cell subtypes in asthma. Thorax 2010; 65:384-90.
Gibson PG, et al. Heterogeneity of airway inflammation in persistent asthma : evidence of neutrophilic inflammation and increased sputum interleukin-8. Chest 2001; 119:1329-36.
Green RH, et al. Asthma exacerbations and sputum eosinophil counts: a randomised controlled trial. Lancet 2002; 30:1715-21.
Hillas G, et al. Exhaled nitric oxide and exhaled breath condensate pH as predictors of sputum cell counts in optimally treated asthmatic smokers. Respirology 2011; 16:811-18.
Ito K, et al. Update on glucocorticoid action and resistance. J Allergy Clin Immunol 2006; 117:522-43.
Nair P, et al. Mepolizumab for Prednisone-Dependent Asthma with Sputum Eosinophilia. N Engl J Med 2009; 360:985-93.
Simpson JL, et al. Inflammatory subtypes in asthma: assessment and identification using induced sputum. Respirology 2006; 11:54-61.

## Claims

1. A method for determining the asthma inflammatory phenotype in a subject suffering from asthma, the method comprising:
(a) determining the expression profile of the genes *CLC, CPA3, DNASE1L3, IL1B*, *ALPL* and *IL8RB,* wherein the expression profile comprises or is based on the expression levels of said genes in a biological sample which has been isolated from the subject, wherein the expression profile of said genes is indicative of the asthma inflammatory phenotype.

2. A method according to claim 1, wherein the expression profile of said genes and the determination of inflammatory phenotype is based on multiple logistic regression analysis of the gene expression levels.

3. A method according to claim 1 or 2, wherein the method enables the determination of whether the subject has eosinophilic asthma or non-eosinophilic asthma.

4. A method according to claim 2, wherein multiple logistic regression analysis of the expression profile of said genes facilitates the distinction between:
(i) eosinophilic asthma and non-eosinophilic asthma, typically where non-eosinophilic asthma is neutrophilic asthma or paucigranulocytic asthma;
(ii) neutrophilic asthma and non-neutrophilic asthma, typically where non-neutrophilic asthma is eosinophilic asthma or paucigranulocytic asthma;
(iii) neutrophilic asthma and paucigranulocytic asthma;
(iv) paucigranulocytic asthma and non-paucigranulocytic asthma, typically where non-paucigranulocytic asthma is neutrophilic asthma or eosinophilic asthma; or
(v) eosinophilic asthma and neutrophilic asthma.

5. A method according to any one of claims 1 to 4, wherein the expression profile of the genes in the sample from the subject is compared to a reference expression profile, wherein the reference expression profile comprises an expression profile of the same genes derived from one or more individuals known not to suffer from asthma, or alternatively known to have a particular asthma inflammatory phenotype.

6. A method according to any one of claims 1 to 5, wherein for determination of the expression profile, expression is measured at the messenger ribonucleic acid (mRNA) level, or at the polypeptide level.

7. A method for distinguishing between eosinophilic asthma and non-eosinophilic asthma in a subject, the method comprising:
(a) determining the expression profile of the genes *CLC*, *CPA3*, *DNASE1L3*, *IL1B*, *ALPL* and *IL8RB*, based on the expression levels of said genes in a biological sample which has been isolated from the subject, wherein the expression profile of said genes is indicative of the subject having eosinophilic asthma or non-eosinophilic asthma.

8. A method for determining the response of a subject having asthma to corticosteroid therapy, the method comprising the steps of:
(a) determining the expression levels of the genes *CLC*, *CPA3*, *DNASE1L3*, *IL1B* and *ALPL* in a biological sample which had been obtained from the subject before a corticosteroid therapy was administered to the subject, and
(b) determining the expression levels of the genes *CLC*, *CPA3*, *DNASE1L3*, *IL1B* and *ALPL* in a second biological sample which has been obtained from the subject after a corticosteroid was administered to the subject;
wherein a reduction in expression of one or more of *CLC*, *CPA3* and *DNASE1L3* and/or an increase in expression of one or more of *IL1B* and *ALPL* in step (b) compared to step (a) is indicative that the subject is responsive to corticosteroid therapy.

9. A method for diagnosing responders to corticosteroid therapy for treating asthma, the method comprising the steps of:
(a) determining the expression profile of the genes *CLC*, *CPA3*, *DNASE1L3*, *IL1B*, *ALPL* and *IL8RB,* wherein the expression profile comprises or is based on the expression levels of said genes in a biological sample which has been obtained from the subject; and
(b) comparing the gene expression profile from the sample with a reference gene expression profile indicative of responsiveness to corticosteroid therapy, and/or to a reference gene expression profile indicative of non-responsiveness to corticosteroid therapy,
wherein similarity in expression profiles between the sample and reference profiles is indicative of the subject being a responder or non-responder to corticosteroid therapy.

10. A method according to claim 9, wherein a reference expression profile indicative of responsiveness to corticosteroid therapy is obtained from one or more individuals responsive to corticosteroid therapy.

11. A method according to claim 9 or 10, wherein a reference expression profile indicative of non-responsiveness to corticosteroid therapy is obtained from one or more individuals non-responsive to corticosteroid therapy.

12. A method according to any one of claims 1 to 11, wherein the biological sample comprises sputum, optionally induced sputum.

13. A method for determining a treatment regime for a subject suffering from asthma, the method comprising determining the asthma inflammatory phenotype in the subject in accordance with a method of one or more of the preceding claims, and selecting an appropriate treatment regime for the subject on the basis of the determination.

## Patentansprüche

1. Verfahren zum Bestimmen des entzündlichen Asthmaphänotyps in einem Subjekt, das an Asthma leidet, wobei das Verfahren Folgendes umfasst:
(a) Bestimmen des Expressionsprofils der Gene CLC; CPA3, DNASE1L3, IL1B, ALPL und IL8RB, wobei das Expressionsprofil die Expressionsniveaus der Gene in einer biologischen Probe, die von dem Subjekt isoliert worden ist, umfasst oder auf diesen basiert, wobei das Expressionsprofil der Gene hinweisend auf den entzündlichen Asthmaphänotyp ist.

2. Verfahren nach Anspruch 1, wobei das Expressionsprofil der Gene und die Bestimmung des entzündlichen Phänotyps auf mehreren logistischen Regressionsanalysen der Genexpressionsniveaus basiert.

3. Verfahren nach Anspruch 1 oder 2, wobei das Verfahren die Bestimmung, ob das Subjekt eosinophiles Asthma oder nicht-eosinophiles Asthma aufweist, ermöglicht.

4. Verfahren nach Anspruch 2, wobei mehrere logistische Regressionsanalysen des Expressionsprofils der Gene die Unterscheidung erleichtert zwischen:
(i) eosinophilem Asthma und nicht-eosinophilem Asthma, normalerweise, wenn nicht-eosinophiles Asthma neutrophiles Asthma oder paucigranulocytisches Asthma ist;
(ii) neutrophilem Asthma und nicht-neutrophilem Asthma, normalerweise, wenn nichtneutrophiles Asthma eosinophiles Asthma oder paucigranulocytisches Asthma ist;
(iii) neutrophilem Asthma und paucigranulocytischem Asthma;
(iv) paucigranulocytischem Asthma und nicht-paucigranulocytischem Asthma, normalerweise, wenn nicht-paucigranulocytisches Asthma neutrophiles Asthma oder eosinophiles Asthma ist; oder
(v) eosinophilem Asthma und neutrophilem Asthma.

5. Verfahren nach einem der Ansprüche 1 bis 4, wobei das Expressionsprofil der Gene in der Probe von dem Subjekt mit einem Expressionsreferenzprofil verglichen wird, wobei das Expressionsreferenzprofil ein Expressionsprofil derselben Gene umfasst, die von einem oder mehrere Individuen, von denen bekannt ist, dass sie nicht an Asthma leiden, oder von denen alternativ bekannt ist, dass sie einen bestimmten entzündlichen Asthmaphänotyp aufweisen, entnommen wurden.

6. Verfahren nach einem der Ansprüche 1 bis 5, wobei für die Bestimmung des Expressionsprofils, die Expression an dem Botenribonukleinsäure(mRNA)niveau oder an dem Polypeptidniveau gemessen wird.

7. Verfahren zum Unterscheiden zwischen eosinophilem Asthma und nicht-eosinophilem Asthma in einem Subjekt, wobei das Verfahren Folgendes umfasst:
(a) Bestimmen des Expressionsprofils der Gene CLC, CPA3, DNASE1L3, IL1B, ALPL und IL8RB, basierend auf den Expressionsniveaus der Gene in einer biologischen Probe, die von dem Subjekt isoliert worden ist, wobei das Expressionsprofil der Gene hinweisend darauf ist, dass das Subjekt eosinophiles Asthma oder nicht-eosinophiles Asthma aufweist.

8. Verfahren zum Bestimmen der Reaktion eines Subjekts mit Asthma auf eine Corticosteroidbehandlung, wobei das Verfahren die folgenden Schritte umfasst:
(a) Bestimmen der Expressionsniveaus der Gene CLC, CPA3, DNASE1L3, IL1B und ALPL in einer biologischen Probe, die von dem Subjekt erhalten worden ist, bevor eine Corticosteroidbehandlung an das Subjekt verabreicht wurde, und
(b) Bestimmen der Expressionsniveaus der Gene CLC, CPA3, DNASE1L3, IL1B und ALPL in einer zweiten biologischen Probe, die von dem Subjekt erhalten worden ist, nachdem ein Corticosteroid an das Subjekt verabreicht wurde;
wobei eine Reduktion in der Expression eines oder mehrerer CLC, CPA3 und DNASE1L3 und/oder einem Anstieg in der Expression eines oder mehrerer IL1B und ALPL im Vergleich von Schritt (b) zu Schritt (a) hinweisend darauf ist, dass das Subjekt auf eine Coritcosteroidbehandlung reagiert.

9. Verfahren zum Diagnostizieren von Respondern für eine Corticosteroidbehandlung zum Behandeln von Asthma, wobei das Verfahren die folgenden Schritte umfasst:
(a) Bestimmen des Expressionsprofils der Gene CLC, CPA3, DNASE1L3, IL1B, ALPL und IL8RB, wobei das Expressionsprofil die Expressionsniveaus der Gene in einer biologischen Probe, die von dem Subjekt erhalten worden ist, umfasst oder auf diesen basiert; und
(b) Vergleichen des Genexpressionsprofils der Probe mit einem Genexpressionsreferenzprofil, hinweisend auf die Reaktionsfähigkeit auf eine Corticosteroidbehandlung und/oder mit einem Genexpressionsreferenzprofil, hinweisend auf keine Reaktionsfähigkeit auf eine Corticosteroidbehandlung, wobei Gleichheit von Expressionsprofilen zwischen der Probe und den Referenzprofilen hinweisend auf das Subjekt als ein Responder oder ein Non-Responder auf Corticosteroidbehandlung ist.

10. Verfahren nach Anspruch 9, wobei ein Expressionsreferenzprofil hinweisend auf die Reaktionsfähigkeit auf Corticosteroidbehandlung von einem oder mehreren Individuen erhalten wird, die auf eine Corticosteroidbehandlung reagieren.

11. Verfahren nach Anspruch 9 oder 10, wobei ein Expressionsreferenzprofil hinweisend auf keine Reaktionsfähigkeit auf eine Corticosteroidbehandlung von einem oder mehreren Individuen erhalten wird, die nicht auf eine Coritcosteroidbehandlung reagieren.

12. Verfahren nach einem der Ansprüche 1 bis 11, wobei die biologische Probe Sputum, optional induziertes Sputum umfasst.

13. Verfahren zum Bestimmen eines Behandlungsablaufs für ein Subjekt, das an Asthma leidet, wobei das Verfahren Bestimmen des entzündlichen Asthmaphänotyps in dem Subjekt nach einem Verfahren nach einem oder mehreren der vorhergehenden Ansprüche, und Auswählen eines angemessenen Behandlungsablaufs für das Subjekt, basierend auf der Bestimmung, umfasst.

## Revendications

1. Procédé de détermination du phénotype inflammatoire de l'asthme chez un sujet souffrant d'asthme, le procédé comprenant l'étape suivante :
(a) déterminer le profil d'expression des gènes *CLC*, *CPA3*, *DNASE1L3*, *IL1B*, *ALPL* et *IL8RB,* le profil d'expression comprenant, ou étant basé sur, les niveaux d'expression desdits gènes dans un échantillon biologique qui a été isolé du sujet, le profil d'expression desdits gènes indiquant le phénotype inflammatoire de l'asthme.

2. Procédé selon la revendication 1, dans lequel le profil d'expression desdits gènes et la détermination du phénotype inflammatoire sont basés sur l'analyse de régression logistique multiple des niveaux d'expression des gènes.

3. Procédé selon la revendication 1 ou 2, le procédé permettant de déterminer si le sujet souffre d'asthme éosinophile ou d'asthme non éosinophile.

4. Procédé selon la revendication 2, dans lequel l'analyse de régression logistique multiple du profil d'expression desdits gènes facilite la distinction entre :
(i) l'asthme éosinophile et l'asthme non éosinophile, l'asthme non éosinophile étant généralement de l'asthme neutrophile ou de l'asthme pauci-granulocytaire ;
(ii) l'asthme neutrophile et l'asthme non neutrophile, l'asthme non neutrophile étant généralement de l'asthme éosinophile ou de l'asthme pauci-granulocytaire ;
(iii) l'asthme neutrophile et l'asthme pauci-granulocytaire ;
(iv) l'asthme pauci-granulocytaire et l'asthme non pauci-granulocytaire, l'asthme non pauci-granulocytaire étant généralement de l'asthme neutrophile ou de l'asthme éosinophile ; ou
(v) l'asthme éosinophile et l'asthme neutrophile.

5. Procédé selon l'une quelconque des revendications 1 à 4, dans lequel le profil d'expression des gènes dans l'échantillon prélevé chez le sujet est comparé à un profil d'expression de référence, dans lequel le profil d'expression de référence comprend un profil d'expression des mêmes gènes provenant d'un ou de plusieurs individus dont on sait qu'ils souffrent d'asthme ou, en variante, dont on sait qu'ils présentent un phénotype inflammatoire de l'asthme particulier.

6. Procédé selon l'une quelconque des revendications 1 à 5 dans lequel, pour déterminer le profil d'expression, l'expression est mesurée au niveau de l'acide ribonucléique messager (ARNm) ou au niveau du polypeptide.

7. Procédé pour distinguer l'asthme éosinophile de l'asthme non éosinophile chez un sujet, le procédé comprenant l'étape suivante :
(a) déterminer le profil d'expression des gènes *CLC*, *CPA3*, *DNASE1L3*, *IL1B*, *ALPL* et *IL8RB,* sur la base des niveaux d'expression desdits gènes dans un échantillon biologique qui a été isolé du sujet, dans lequel le profil d'expression desdits gènes indique le sujet souffrant d'asthme éosinophile ou d'asthme non éosinophile.

8. Procédé de détermination de la réponse d'un sujet souffrant d'asthme à la corticothérapie, le procédé comprenant les étapes suivantes :
(a) déterminer les niveaux d'expression des gènes *CLC*, *CPA3*, *DNASE1L3*, *IL1B* et ALPL dans un échantillon biologique qui a été obtenu chez le sujet avant qu'une corticothérapie ait été administrée au sujet, et
(b) déterminer les niveaux d'expression des gènes *CLC*, *CPA3*, *DNASE1L3*, *IL1B* et ALPL dans un deuxième échantillon biologique qui a été obtenu chez le sujet après qu'un corticostéroïde a été administré au sujet ; dans lequel une réduction de l'expression d'un ou de plusieurs gènes parmi *CLC*, *CPA3* et *DNASE1L3* et/ou une augmentation de l'expression *d'IL1B* et/ou d'*ALPL* à l'étape (b) par rapport à l'étape (a) indique que le sujet réagit à la corticothérapie.

9. Procédé de diagnostic de répondeurs à la corticothérapie pour traiter l'asthme, le procédé comprenant les étapes suivantes :
(a) déterminer le profil d'expression des gènes *CLC*, *CPA3*, *DNASE1L3*, *IL1B*, *ALPL* et *IL8RB*, dans lequel le profil d'expression comprend ou est basé sur les niveaux d'expression desdits gènes dans un échantillon biologique qui a été obtenu chez le sujet ; et
(b) comparer le profil d'expression des gènes de l'échantillon à un profil d'expression des gènes de référence indique une réactivité à la corticothérapie, et/ou à un profil d'expression des gènes de référence indiquant une non réactivité à la corticothérapie, dans lequel la similarité des profils d'expression entre l'échantillon et les profils de référence indique que le sujet répond ou non à la corticothérapie.

10. Procédé selon la revendication 9, dans lequel un profil d'expression de référence indiquant une réactivité à la corticothérapie est obtenu chez un ou plusieurs individus réagissant à la corticothérapie.

11. Procédé selon la revendication 9 ou 10, dans lequel un profil d'expression de référence indiquant une non-réactivité à la corticothérapie est obtenu chez un ou plusieurs individus ne réagissant pas à la corticothérapie.

12. Procédé selon l'une quelconque des revendications 1 à 11, dans lequel l'échantillon biologique comprend l'expectoration et facultativement l'expectoration induite.

13. Procédé de détermination d'un régime de traitement pour un sujet souffrant d'asthme, le procédé comprenant la détermination du phénotype inflammatoire de l'asthme chez le sujet conformément à un procédé selon une ou plusieurs des revendications précédentes et la sélection d'un régime de traitement approprié pour le sujet sur la base de la détermination.
